# EUROPÄISCHE PATENTSCHRIFT

(11) **EP 0 577 143 B1**
(45) Veröffentlichungstag und Bekanntmachung des Hinweises auf die Patenterteilung: **27.01.1999**
(21) Anmeldenummer: 93110716.3
(22) Anmeldetag: 05.07.1993
(51) Int. Cl.: A61K 47/42

(54) **Feste und flüssige Lösungen von Flavonoiden**
Solid and liquid solutions of flavonoids
Solutions solides et liquides de flavonoides

(30) Priorität: 03.07.1992 DE 4221879; 03.07.1992 DE 4221835; 03.07.1992 DE 4221834
(43) Veröffentlichungstag der Anmeldung: 05.01.1994
(73) Patentinhaber: ALFATEC-PHARMA GmbH, 69120 Heidelberg (DE)
(72) Erfinder: Wunderlich, Jens-Christian, 69126 Heidelberg (DE); Pabst, Joachim, Dr., 64354 Reinheim (DE); Schick, Ursula, 69198 Schriesheim (DE); Werry, Jürgen, Dr., 67071 Ludwigshafen (DE); Freidenreich, Jürgen, Dr., 69198 Schriesheim (DE)
(74) Vertreter: Winter, Brandl, Fürniss, Hübner, Röss, Kaiser, Polte, Kindermann Partnerschaft

(56) Entgegenhaltungen:
- WO-A-92/02206
- GB-A- 1 072 263
- DATABASE WPI Section Ch, Week 9321, Derwent Publications Ltd., London, GB; Class D13, AN 93-174355 & SU-A-1 738 215 (GOSAGROPROM SCI PRODN ASSOC) 7. Juni 1992
- DATABASE WPI Section Ch, Week 9108, Derwent Publications Ltd., London, GB; Class B02, AN 91-055557 & JP-A-3 007 224 (TSUMURA & CO) 14. Januar 1991
- DATABASE WPI Section Ch, Week 8917, Derwent Publications Ltd., London, GB; Class B04, AN 89-128083 & RO-A-95 618 (CENT CERC BIOLOGICE) 30. September 1988

## Beschreibung

Die vorliegende Erfindung betrifft eine Flavonoid-Zubereitung, die wenigstens ein Flavonoid in molekulardisperser Verteilung in einer Basissubstanz aus einem hydrophilen Peptid mit einem Molekulargewicht über 100 D enthält. Die Erfindung betrifft ebenfalls ein Verfahren zur Herstellung einer solchen Flavonoid-Zubereitung sowie pharmazeutische und kosmetische Zubereitungen oder diätetische Lebensmittel, die solche Flavonoid-Zubereitungen enthalten.

Die Stoffklasse der Flavonoide, worunter auch die Flavonglykoside und ihre zugehörigen Aglyka fallen, insbesondere vertreten durch das wichtigste Flavonglykosid Rutin, steht seit vielen Jahren im Widerstreit der Wissenschaft als vitaminähnliche Stoffe (Vitamin P). Der ursprüngliche Stellenwert der Flavonglykoside, besonders der des Rutins, in der Behandlung von peripheren Durchblutungsstörungen mußte der Erkenntnis weichen, daß die isolierten Flavonglykoside als Reinstoff nach oraler Verabreichung in so geringem Umfang resorbiert werden, daß bei den angewendeten Dosierungen von ihnen kaum noch eine klinisch relevante Wirkung ausgehen kann. Ihre u.a. durchblutungsfördernde Wirkung konnte bisher nur dann beobachtet werden, wenn sie an biologische Strukturen gebunden waren und so als Lebensmittel in den Körper gelangten.

Alle Versuche für die isolierten Flavonglykoside eine resorptionsverbessernde Formulierung zu entwickeln, verfehlten bisher ihr Ziel. Auch moderne galenische Verfahren der Her-stellung von Einschlußverbindungen mit zyklischen Oligosacchariden wie Cyclodextrinen haben keine Resorptionsverbesserungen ergeben. Die Löslichkeitseigenschaften der Flavonglykoside, aber auch die der freien Flavone, erlaubten bisher eine Löslichmachung in wässrigen Systemen als wichtigste Voraussetzung für eine verbesserte enterale Resorption nicht.

Es ist leicht verständlich, daß bei der Komplexität der Inhaltsstoffe von flavonoidhaltigen Pflanzen solche mit hydrophilem Charakter (leicht bzw. ausreichend wasserlösliche), als auch solche mit lipophilem Charakter (nicht bzw. schwer wasserlöslich) zugegen sein können, zumal bei der Extraktgewinnung aus den betreffenden Pflanzenteilen meist organische Lösungsmittel wie z.B. Alkohole im Gemisch mit Wasser benutzt werden.

So müssen flüssige Arzneizubereitungen wie z.B. Tropfen zum Einnehmen oder Injektionslösungen spezielle Hilfsstoffe (Lösungsvermittler) enthalten und können nicht Wasser als alleiniges Lösungsmittel enthalten. In Wasser wäre stets nur ein verschwindend geringer Teil an Flavonglykosiden oder ihren Aglyka löslich. Lipophile Inhaltsstoffe, die am Zustandekommen des pharmakologischen Potentials des Pflanzenextrakts einen entscheidenden Anteil haben können, würden für eine arzneiliche Wirkung verloren gehen bzw. stünden dem Organismus nicht zur Resorption zur Verfügung.

Bei sauren oder basischen lipophilen Komponenten kann z.B. versucht werden, eine stabile, wasserlösliche Salzform zu finden. Wenn dies nicht möglich ist, kann durch Zusatz geeigneter, wassermischbarer organischer Lösungsmittel, beispielsweise Ethanol, 1,2-Propylenglykol, Glycerol oder Polyethylenglykolen die Löslichkeit von lipophilen Stoffen in Wasser erhöht werden. Nach diesem Prinzip sind handelsübliche, flüssige, Flavonoide enthaltende Arzneizubereitungen zur oralen bzw. peroralen Einnahme aufgebaut.

Es wäre grundsätzlich falsch, von diesen technologischen Maßnahmen her bzw. vom Vorliegen in Lösung der Inhaltsstoffe dieser Pflanzenextrakte in der flüssigen Arzneizubereitung Rückschlüsse auf die Resorption im Organismus vornehmen zu wollen.

Nach der Theorie der passiven Diffusion werden Arzneistoffe im Gastrointestinaltrakt nur in gelöster Form resorbiert. Um daher in vivo eine Resorption von Flavonoiden sicherzustellen und damit einen optimalen pharmakologischen bzw. therapeutischen Effekt zu erzielen, muß mindestens der gelöste Zustand dieser Flavonoide - nicht nur in der Zubereitung, sondern auch in vivo - gewährleistet sein.

Dies ist jedoch bei einer Arzneizubereitung, die organische Lösungsmittel bzw. Salze des Rutins enthält, nicht der Fall, wie man sich leicht vorstellen kann. Durch physiologische Einflüsse (Verdünnungseffekte oder pH-Verschiebungen) im Magen-Darm-Trakt wird die erzwungene in vitro-Lösungsvermittlung rückgängig gemacht und als Folge drohen sofortige Ausfällungen oder gröbere Ausflockungen.

Eigene Untersuchungen an handelsüblichen, Flavonoide enthaltenden, flüssigen Arzneizubereitungen (Tropfen) zeigten jedoch überraschenderweise, daß sofort gröbere Ausflockung von Inhaltsstoffen auftritt, sobald eine übliche Tropfendosis in Wasser bzw. künstlichen Magensaft gelangt. Daher dürfen an der vollständigen in vivo-Resorbierbarkeit der Wirksubstanzen solcher Zubereitungen berechtigte Bedenken angemeldet werden.

Ähnlich ist die Situation bei den handelsüblichen, festen peroralen Darreichungsformen (z.B. Tabletten, Dragees) zu beurteilen. Obwohl Flavonglykoside oder ihre Aglyka meist durch Verarbeitung mit Polyethylenglykol als lösungsvermittelndem Hilfsstoff in die feste Darreichungsform überführt werden, kann analog keine ausreichende Löslichkeit der Flavonglykoside oder ihrer Aglyka unter physiologischen Bedingungen erwartet werden.

Die Herstellung von Flavonglykosid-Derivaten, z.B. Rutinschwefelsäure-ester, führte zwar zur Verbesserung der Löslichkeit in wässrigen Systemen; die Stabilität gegen Hydrolyse dieser Ester im Magen-Darm-Trakt ist aber so gering, daß ihre Verseifung unverzüglich ohne Chance einer verbesserten Resorption eintritt. Die einzig bisher erfolgreiche Verbesserung der Resorption gelang durch Herstellung von Hydroxy-ethyl-Verbindungen. Die Gruppe der Flavonglykosid-hydroxy-ethyl-äther (z.B. Tris-, Di-, und Mono-hydroxy-ethyl-rutin) liegt in der Regel als unterschiedliche Mischung der verschiedenen Derivate (z.B. Troxerutin) vor, sind also keine Reinstoffe mehr. Die verbesserte Resorption dieser Verbindungen führte aber zu einem wesentlichen Verlust der wertvollen pharmakologischen Eigenschaften der Flavonglykoside bzw. ihrer Aglyka als die eigentlichen Träger der pharmakologischen Wirkung.

Troxerutin und andere Flavonglykosid-Konjugate haben jedoch die ursprüngliche Rolle der isolierten Flavonglykoside, insbesondere die des Rutins, in der Therapie von peripheren Durchblutungsstörungen übernommen. Der noch immer fehlende Nachweis für eine enterale Resorption der Flavonglykoside als Reinstoff hat sogar dazu geführt, daß sie ihren Status als zugelassenes Arzneimittel verlieren werden. Die hier anzumeldenden pharmazeutischen Zubereitungen, sowie Verfahren der Löslichmachung von Flavonglykosiden würden in Anbetracht der vielfältigen vergeblichen Versuche eine kaum noch erwartete tete Innovation bedeuten. In Magensaft-stabiler Form löslich gemachte und damit resorbierbar gemachte Flavonglykoside könnten eine außerordentliche wirtschaftliche Bedeutung als bestens verträgliche Naturheilmittel für eine Reihe von Indikationen erlangen.

Der im Prüfungsverfahren der vorliegenden Patentanmeldung aufgefundene Stand der Technik hat mit der vorliegenden Erfindung nur scheinbare Gemeinsamkeiten:

WO-A-92 02206 betrifft Blätter aus gefriergetrocknetem nativem Kollagen, die kosmetische Formulierungen zur Behandlung von Couperose enthalten. Bezüglich der Blätter aus nativem Kollagen wird auf die EP 0284789 und parallele US- bzw. FR-Patentschriften verwiesen. Wie man der EP 0284789 entnehmen kann, ist die dort offenbarte Herstellung von Kollagenblättern für die vorliegende Anmeldung nicht relevant.

GB-A-1 072 263 beschreibt ein therapeuthisches Mittel gegen Erkältung. Es enthält keine molekulardisperse Verteilung von Flavonoid in einer Basissubstanz, z. B. Gelatine.

Derwent 93-174 355 entsprechend der SU-A-1 738 215 beschreibt einen Kräutertee und seine Herstellung. Dazu werden getrocknete, gemahlene Kräuter und Zucker mit 20%-iger wässriger Gelantinelösung gemischt, granuliert und getrocknet. Die gemahlenen Kräuter sind offensichtlich nicht gelöst; daher liegt keine molekulardisperse Verteilung vor.

Derwent 91-055 557 betrifft Mittel gegen Retroviren zur Behandlung von Brustkrebs usw. Diese können oral oder parenteral gegeben werden und enthalten als Wirkstoff ein Flavonoid, z. B. Rutin in Bindemittel, z. B. Gelatine. Daß Flavonoid in molekulardisperser Verteilung in dem Bindemittel, z. B. Stärke, Dextrin, Gummi arabicum oder Gelatine vorliegt, wird nicht näher erläutert. Außerdem enthalten die Mittel Disintegratoren, oberflächenaktive Substanzen, Gleitmittel und Fluidisierungsmittel.

Derwent 89-128 083 beschreibt Suppositorien, die Trockensubstanz von Spirulina Pratensis und Flavonoidextrakt von Propolis in üblichen Hilfsmitteln, z. B. Kakaobutter oder Glycerin-Gelatine, enthalten. Aus dieser Literaturstelle geht nicht hervor, daß der Flavonoid-Extrakt in Gelatine gelöst, d.h. molekulardispers verteilt vorliegt.

Der Erfindung liegt daher die Aufgabe zugrunde, Flavonoide in eine solche Zubereitung zu überführen, daß der gelöste (molekulardisperse) Zustand des Flavonoids auf einfache Weise, ohne an die Anwesenheit organischer Lösungsmittel oder unerwünschte Nebenwirkungen verursachende Lösungsvermittler direkt gebunden zu sein, sowohl beim Lagern als auch bei der Anwendung der Zubereitung durch den Verbraucher bzw. Patienten gewährleistet ist.

Aufgabe der vorliegenden Erfindung ist es weiterhin insbesonders, Flavonoide enthaltende Arzneimittel zu entwickeln, die die zum Stand der Technik genannten Nachteile vermeiden und daher die Wirksamkeit bzw. Bioverfügbarkeit solcher Stoffe verbessern, um eine effektive therapeutische Nutzung derartiger Phytopharmaka zu erreichen.

Diese Aufgabe wird erfindungsgemäß durch eine Flavonoid-Zubereitung gelöst, die wenigstens ein Flavonoid in molekulardisperser Verteilung in einer Basissubstanz aus einem hydrophilen Peptid mit einem Molekulargewicht über 100 D enthält.

Die Aufgabe wird weiterhin durch ein Verfahren zur Herstellung einer solchen Flavonoid-Zubereitung gelöst. Ausführungsformen der erfindungsgemäßen Zubereitungen sowie des Verfahrens zu ihrer Herstellung werden in den Unteransprüchen genannt und beansprucht.

Schließlich wird diese Aufgabe durch pharmazeutische oder kosmetische Zubereitungen oder diätetische Lebensmittel gelöst, die solche Flavonoid-Zubereitungen enthalten.

Die vorliegende Erfindung beruht nun auf der völlig überraschenden Feststellung, daß allein eine Basissubstanz aus einem hydrophilen Peptid mit einem Molekulargewicht über 100 D ausreichend ist, Flavonoide in gelöster Form so zu stabilisieren, daß keine organischen Lösungsmittel oder Tenside etc. mehr als Lösungsvermittler notwendig sind.

Derartige erfindungsgemäße Systeme können als flüssige, wäßrige Lösungen vorliegen, aber es kann auch durch geeignete Verfahren das Lösungsmittel entzogen werden (z.B. Sprüh- oder Gefriertrocknung). Die so getrockneten Produkte lassen sich durch Wasserzusatz bzw. im physiologischen Milieu wieder zu ihrem Ausgangszustand auflösen (redispergieren).

Der Begriff "Basissubstanz aus einem hydrophilen Peptid mit einem Molekulargewicht über 100 D" bedeutet hier eine aus Aminosäuren oder Derivaten von Aminosäuren aufgebaute Substanz, wie beispielsweise eine von Kollagen abgeleitete Substanz, ausgewählt aus der Gruppe bestehend aus: Gelatine, fraktionierte Gelatine, Kollagenhydrolysate, Gelatinederivate; sowie deren Mischungen.

Eine bevorzugte Ausführungsform der Erfindung betrifft den Extrakt aus Gingko biloba, dessen aktives Prinzip vorwiegend aus Flavonoiden besteht.

Zur Herstellung von Phytopharmaka verwendete Pflanzenextrakte, wie beispielsweise der aus den Blättern des Baumes Ginkgo biloba (Linné) gewonnene Ginkgo biloba-Extrakt, enthalten oft eine Komplexität von Inhaltsstoffen, die in vielen Fällen sogar noch nicht vollständig aufgeklärt ist.

So wurden in Ginkgo biloba-Blättern bis jetzt über 2000 Inhaltsstoffe nachgewiesen. Ginkgo biloba-Extrakte enthalten immerhin noch etwa 200 Inhaltsstoffe.

Man ist heute überwiegend der Überzeugung, daß die therapeutische Wirksamkeit solcher Phytopharmaka wohl nicht auf einzelne Inhaltsstoffe zurückzuführen ist, sondern daß das Zusammenwirken einer Vielzahl von verschiedenen Komponenten letztlich erst das arzneiliche Wirkprofil ausmacht.

Ginkgo biloba-Extrakt enthaltende Phytopharmaka sind z.B. bei verschiedenen ischämischen Erkrankungen indiziert. Sie fördern die Durchblutung bei minimalen kardialen Wirkungen und hemmen die Thrombozytenaggregation. Ferner üben sie eine Schutzfunktion bei Hypoxieschäden des Gewebes aus, wirken protektiv und kurativ bei Hirnödemen, Myelinschäden und radikalinduzierten pathologischen Veränderungen.

Eine grobe Klasseneinteilung der Inhaltsstoffe von Ginkgo biloba-Extrakt ergibt sich, wenn man zwischen
a) einer Flavonglykosidfraktion (z.B. Ginkgoflavonglykoside) und
b) einer Nichtflavonfraktion (z.B. Terpenlactone) unterscheidet.

Eine weitere bevorzugte Ausführungsform der Erfindung betrifft eine pharmazeutische Zubereitung mit der Wirkung des Gingko biloba-Extraktes. Bei klinischen Studien zur Resorption von Rutin, das molekulardispers in Gelatine verteilt von den Probanden eingenommen worden war, wurde nun überraschend festgestellt, daß Rutin in vivo die Metaboliten Quercetin, Kämpferol und Isorhamnetin bildet. Die Metaboliten von Rutin sind daher identisch mit den wichtigsten Wirkstoffen des Gingko biloba-Extrakts.

Der vorliegenden Erfindung in einer weiteren bevorzugten Ausführungsform liegt daher die Aufgabe zugrunde, die Wirkstoffe von Gingko biloba aus einer Rohstoffquelle, die leichter zugänglich ist als ein Gingko biloba-Extrakt in einer für den Patienten therapiegerechten und in ausreichendem Maße aufnehmbaren Darreichungsform zur Verfügung zu stellen.

Diese Aufgabe wird erfindungsgemäß durch eine pharmazeutische Zubereitung gelöst, die Rutin in molekulardisperser Verteilung in einer Basissubstanz aus einem hydrophilen Peptid mit einem Molekulargewicht über 100 D neben pharmazeutisch üblichen Träger- und Hilfsstoffen enthält.

Unter einer pharmazeutischen Zubereitung im Sinne der Erfindung werden sowohl Arzneimittelzubereitungen, als auch diätetische Lebensmittelzubereitungen (health care) verstanden.

Rutin ist ein Glycosid des Quercetins mit Rutinose, das in vielen Pflanzenarten vorkommt und daher wesentlich leichter zugänglich ist als der Extrakt aus Gingko biloba. Solche Pflanzenarten kommen aus Gattungen wie Fagopyrum, Sophora, Viola, Ruta, Solidago, Verbascum usw.

Die obengenannte Aufgabe wird ferner durch ein Verfahren zur Herstellung einer pharmazeutischen Zubereitung mit der Wirkung des Gingko biloba-Extrakts gelöst, das dadurch gekennzeichnet ist, daß man Rutin in Lösung überführt und diese Lösung mit einer wäßrigen Lösung einer Basissubstanz aus einem hydrophilen Peptid mit einem Molekulargewicht über 100 D vermischt und das oder die Lösungsmittel entfernt, wodurch man eine molekulardisperse Verteilung von Rutin erhält, wobei bei man in der ersten oder nach der zweiten Stufe gegebenenfalls pharmazeutisch übliche Träger- und Hilfsstoffe zusetzt.

Die oben genannte Aufgabe wird auch durch die Verwendung einer erfindungsgemäßen Flavonoid-Zubereitung, die Rutin als Flavonoid enthält, zur Herstellung eines Arzneimittels zur Erzielung der pharmakologischen Wirkung von Quercetin, Kämpferol und/oder Isorhamnetin, d.h. zur Erzielung der wichtigsten pharmakologischen Wirkungen des Gingko biloba-Extraktes gelöst.

Erfindungsgemäß wird nicht nur der Vorteil erreicht, daß die Wirkstoffe des Gingko biloba-Extrakts wesentlich leichter als nach dem Stand der Technik verfügbar sind. Ebenso bedeutsam ist, daß die Wirksamkeit bzw. Bioverfügbarkeit der Wirkstoffe des Gingko biloba-Extrakts erfindungsgemäß verbessert wird und damit eine effektive therapeutische Nutzung der Wirkstoffe des Gingko biloba-Extrakts erreicht wird.

Damit wird mit einer erfindungsgemäßen Rutin-Zubereitung ein Indikationsspektrum zugänglich, wie es bisher für die Zubereitungen des Gingko biloba-Extrakts bekannt ist. Unter Gingko biloba-Extrakt im Sinne der Erfindung werden handelsübliche, Gingko biloba-Extrakt enthaltende Arzneimittel bzw. handelsübliche Gingko biloba-Extrakte verstanden.

Indikation bzw. Wirksamkeit von Gingko biloba-Extrakt, insbesondere der in Gingko biloba-Extrakt enthaltenen Flavonglykosidfraktion, kann beispielsweise vorliegen bei:
- ischämischen Erkrankungen
- zentralen und peripheren arteriellen Durchblutungsstörungen
- Gefäßstörungen verschiedener Genese
- Neuropathien
- Encephalopathien
- Myelopathien
- Neigung zu Thrombozytenaggregation
- Hypoxieschäden
- radikalinduzierten, pathologischen Veränderungen
- Hirnleistungsstörungen
- Hirnödemen
etc.

Gemäß der weiteren Ausführungsform der vorliegenden Erfindung ist eine Basissubstanz aus einem hydrophilen Peptid mit einem Molekulargewicht über 100 D ausreichend, Rutin in gelöster Form so zu stabilisieren, daß keine organischen Lösungsmittel oder Tenside usw. mehr als Lösungsvermittler notwendig sind.

Gelatine ist ein aus kollagenhaltigem Material gewonnenes Skleroprotein, das je nach Herstellungsprozeß unterschiedliche Eigenschaften hat. Sie besteht im wesentlichen aus vier Molekulargewichtsfraktionen, die die physikalisch-chemischen Eigenschaften in Abhängigkeit vom Molekulargewicht und prozentualem Gewichtsanteil beeinflussen. Je höher z.B. der Anteil Mikrogel (10⁷ bis 10⁸ D) liegt, desto höher ist auch die Viskosität der wäßrigen Lösung. Handelsübliche Sorten enthalten bis zu 15 Gewichtsprozent. Die Fraktionen der alpha-Gelatine und deren Oligomere (9,5 x 10⁴ / 10⁵ bis 10⁶ D) sind entscheidend für die Gelfestigkeit und liegen üblicherweise zwischen 10 und 40 Gewichtsprozent. Molekulargewichte unterhalb der alpha-Gelatine werden als Peptide bezeichnet und können in herkömmlichen Gelatinequalitäten (niedrigbloomig) bis zu 80 Gewichtsprozent betragen.

Gelatine besitzt ein temperatur- und konzentrationsabhängiges Sol-Gel-Umwandlungsverhalten, das von der molekularen Zusammensetzung abhängig ist. Als Konventionsmethode für das Gelbildungsvermögen wird die Bloomzahl angegeben. Niedrige Handelsqualitäten beginnen bei 50 Bloom, hochbloomige Sorten liegen bei etwa 300 Bloom.

Die chemischen und physikalischen Eigenschaften von Gelatine variieren je nach Herstellungsverfahren, wobei besonders schonend gewonnene Gelatinesorten (geringer Anteil an rechtsdrehenden Aminosäuren und Peptiden) kurze Sol-Gel-Umwandlungsgeschwindigkeiten und Schmelzpunkte oberhalb 37°C (gemessen als 10%ige Lösung) aufweisen.

Fraktionierte Gelatine stellt den Spezialfall von Gelatine dar und wird durch spezielle Herstellungstechniken, wie z.B. Ultrafiltration aus herkömmlicher Gelatine gewonnen. Die Zusammensetzung kann z.B. durch Entfernung von Peptiden (MG < 9,5 x 10⁴ D) oder durch Mischungen aus Einzelfraktionen wie z.B. alpha-Ketten, dimeren und trimeren Ketten oder Mikrogel varriiert werden.

Gelatinederivate sind chemisch veränderte Gelatinen, wie z.B. succinylierte Gelatine, die auch als Plasmaexpander bekannt sind.

Kollagen in nativer Form ist wasserunlöslich. Durch spezielle Herstellungsverfahren gibt es heute lösliche Kollagentypen mit einem durchschnittlichen Molekulargewicht von ca. 300.000 D.

Unter Kollagenhydrolysat wird ein von Kollagen oder Gelatine druckhydrolytisch oder enzymatisch gewonnenes Produkt ohne Gelbildungsvermögen verstanden. Die Molekulargewichtszusammensetzung kann herstellungsbedingt zwischen einigen Hundert D bis unterhalb von 9,5 x 10⁴ D liegen. Kollagenhydrolysate sind kaltwasserlöslich.

Neuentwickelte Produkte stellen die Pflanzenproteine und deren Hydrolysate dar, die in ihren Eigenschaften weitgehend den Kollagenhydrolysaten entsprechen. Sie werden vorzugsweise aus Weizen und Soja gewonnen und besitzen beispielsweise Molekulargewichte von 200.000-300.000 D bzw. 1.000-10.000 D.

Elastinhydrolysate werden enzymatisch aus Elastin gewonnen und bestehen aus einer einzigen Polypeptidkette mit einem hohen Anteil an nichtpolaren Aminosäuren. Sie können daher auch in lipophilen Systemen verwendet werden. Elastinhydrolysate weisen ein Molekulargewicht von 2.000 - 3.000 D auf und sind auf der Haut stark filmbildend.

Es hat sich weiterhin gezeigt, daß die Verwendung einer von Kollagen abgeleiteten Substanz aus der oben erwähnten Gruppe als lösungsvermittelnde Basissubstanz für Flavonoide nicht nur den Vorteil einer Stabilisierung solcher Stoffe in wäßriger Lösung bietet, sondern darüberhinaus noch weitreichenderen Nutzen aufweist.

Die Flavonoide liegen in einer erfindungsgemäßen Zubereitung einerseits geschützt vor äußeren Einflüssen wie z.B. Licht, Luftsauerstoff etc. vor. Liegt die erfindungsgemäße Zubereitung in fester, wiederauflösbarer Form vor, ist darüberhinaus ein Schutz empfindlicher Inhaltsstoffe vor Feuchtigkeit bzw. Feuchtigkeitszutritt gewährleistet. Damit sind empfindlichen Pflanzeninhaltsstoffe (Flavonoide) in ihrer galenischen Zubereitung gleichzeitig wirksam vor Aktivitätsverlusten durch Zersetzungsprozesse bewahrt.

Weiterhin können von Kollagen abgeleitete Substanzen vorteilhafterweise einen unangenehmen, z.T. bitteren Geschmack von Flavonoiden, wie im Falle von Rutin, in einer erfindungsgemäßen Zubereitung überdecken.

In Bezug auf Resorption bzw. Bioverfügbarkeit der Flavonoide aus einer erfindungsgemäßen pharmazeutischen Zubereitung haben eigene Untersuchungen gezeigt, daß eine gesteigerte Resorption und damit verbunden eine signifikante Erhöhung der Bioverfügbarkeit gegenüber herkömmlichen Zubereitungen bei der Applikation in vivo zu erreichen ist.

Die Anwesenheit der von Kollagen abgeleiteten Substanzen, wie z.B. Gelatine als Makromolekül primär biogenen Ursprungs stellt dabei die größtmögliche Verträglichkeit der Arzneizubereitung im Organismus sicher. Unerwünschte Nebenwirkungen von sonstigen Hilfsstoffen, z.B. Irritationen von Schleimhäuten, wie sie etwa durch organische Lösungsmittel oder Tenside hervorgerufen werden können, fallen so ganz weg oder können zumindest entscheidend reduziert werden.

Damit stellt die vorliegende Erfindung einen überaus wertvollen Beitrag zur sinnvollen therapeutischen Nutzung und Anwendung von Flavonoiden enthaltenden Phytopharmaka dar.

Unter Flavonoiden im Sinne der vorliegenden Erfindung versteht man Flavonglykoside oder ihre Aglyka aus flavonoidhaltigen Pflanzen oder Pflanzenteilen, wie z.B. Blätter, Blüten Früchten, Samen, Rinde, Wurzeln, Kraut, Knospen etc., die frisch oder getrocknet oder auch gepulvert sein können.

Insbesondere kann es sich um Inhaltsstoffe aus Pflanzen oder Pflanzenteilen der folgenden Gattungen, wie z.B. Arnica, Citrus, Betula, Crataegus, Fagopyrum, Glycyrrhiza, Ononis, Sambucus, Silybum, Sophora, Helichrysum, Tilia, Verbascum, Viola, Filipendula, Prunus, Robinia, Calendula, Juglans, Ruta, Polygonum, Solidago, Anthemis, Cereus, Tussilago, Chamomilla, Primula, Potentilla, Equisetum, Herniaria, Leonurus, Sarothamnus, Veronica, Eriodictyon, Orthosiphon, Carthamus, Lespedeza, Passiflora, Aesculus, Melissa, Hypericum und andere handeln.

Weiterhin umfaßt der Begriff "Flavonoide" einzelne aus diesen Pflanzen oder Pflanzenteilen extrahierte bzw. isolierte Flavonoide, deren Glykoside oder Aglyka, deren Derivate (Ether, Ester, Komplexe, Salze, Hydrolyseprodukte u.a. chemische Molekülvariationen) sowie auch synthetisch gewonnene Flavonoide, oder Mischungen der vorgenannten Substanzen.

An einzelnen Stoffen sind beispielhaft Substanzen mit folgenden gemeinsamen Strukturen zu nennen:
1. Aus der Flavonreihe:
   z.B. die Glykoside Vitexin, Orientin und Diosmin mit den Aglyka Apigenin, Luteolin und Diosmetin;
2. Aus der Flavonol-Reihe:
   z.B. die Glykoside Hyperosid, Isoquercitrin, Quercitrin, Hyperosid, Rutin mit dem Aglykon Quercetin, Astragalin mit dem Aglycon Kämpferol, sowie die Aglyka Myricetin, Isorhamnetin, Rhamnetin;
   weiterhin der hauptsächlich aus Silybin, Dehydrosilybin, Silydianin, Silychristin und Silybinpolymeren bestehende Wirkstoffkomplex Silymarin;
3. Aus der Flavanonol-Reihe:
   z.B. die Glykoside Naringin, Hesperidin, Neohesperidin, Eriocitrin, mit den Aglyka Naringenin, Hesperetin, Eriodictyol, sowie die weiteren Aglyka Homoeriodictyol, Taxifolin, Liquiritigenin etc.

Spezielle entferntere Flavonoidabkömmlinge wie z.B. Proanthocyanidine, Catechine, Isoflavone, Rotenoide etc. können als Pflanzeninhaltsstoffe in besonderen Spezies (z.B. Isoflavone häufig in Vertretern der Fabaceen) enthalten sein.

Andere Flavonoidderivate gewinnt man durch Ringöffnung im Alkalischen und nachfolgende Hydrierung. Aus den Citrusbioflavonoiden Naringin und Neohesperidin erhält man so z.B. die stark süß schmeckenden Dihydrochalkonglykoside. Diese Stoffe können als Süßungsmittel eingesetzt werden.

Bei Ginkgo biloba-Extrakt bzw.-Extraktbestandteilen, die extrem thermolabil sind, ermöglicht die Erfindung in einer weiteren Ausführungsform vorteilhafterweise, Zubereitungen mit erfindungsgemäßen Eigenschaften zur Verfügung zu stellen, die ausschließlich bei Raumtemperatur, d.h. ohne Anwendung von Wärme hergestellt sind, z.B. durch Lyophilisation. Bei dieser Vorgehensweise kann man ein hydrophiles Peptid, ausgewählt aus der Gruppe bestehend aus: Pflanzenproteine, Pflanzenproteinhydrolysate, Elastinhydrolysate, Kollagenhydrolysate, kaltwasserlösliche Gelatine, Gelatinederivate; sowie deren Mischungen; mit einem Maximum der Molekulargewichtsverteilung unterhalb 10⁵ D, verwenden.

Solche erfindungsgemäßen Zubereitungen zeigen in kaltem Wasser eine sehr schnelle bzw. beschleunigte Wiederauflösung, in der Regel innerhalb von 2 min, und können daher vorteilhaft zur Herstellung von Instant-Zubereitungen, Parenteralia etc. verwendet werden.

Die oben erwähnten hydrophilen Peptide ermöglichen ebenfalls, aufgrund ihrer guten Eigenschaften der Verpreßbarkeit, eine Direkttablettierung einer erfindungsgemäßen Zubereitung.

Weiterhin können hydrophile Peptide, insbesondere von Kollagen abgeleitete Substanzen vorteilhafterweise einen unangenehmen Geschmack des Ginkgo biloba-Extrakts in einer erfindungsgemäßen Zubereitung überdecken. Diese Geschmacksüberdeckung kann noch verstärkt werden durch Zusatz von Sorbitol, der neben seiner Eigenschaft als Weichmacher vorteilhaft die Funktion eines Süßstoffs mit nicht-kariogener Eigenschaft besitzt.

In Bezug auf Resorption bzw. Bioverfügbarkeit des Ginkgo biloba-Extrakts aus einer erfindungsgemäßen Zubereitung haben eigene Untersuchungen gezeigt, daß eine gesteigerte Resorption und damit verbunden eine signifikante Erhöhung der Bioverfügbarkeit gegenüber herkömmlichen Zubereitungen bei der Applikation in vivo zu erreichen ist.

Die gesteigerte Resorptionsrate des Ginkgo biloba-Extrakts aus einer erfindungsgemäßen Zubereitung läßt sich sehr eindrucksvoll zeigen, wenn man beispielsweise den peroralen Applikationsweg einer pharmazeutischen Zubereitung betrachtet:

Nach Wiederauflösen einer erfindungsgemäßen Zubereitung im physiologischen Milieu bleibt die molekulardisperse Verteilung des Ginkgo biloba-Extrakts erstaunlicherweise erhalten. Ausfällungen bzw. Ausflockungen werden durch die mit den zu resorbierenden Substanzen gebildeten inter- und intramolekularen Konjugate bzw. Einlagerungen aus Gelatine bzw. einer von Kollagen abgeleiteten Substanz, wirksam verhindert. Weiterhin schützt die umhüllende (viskose) Solschicht den Ginkgo biloba-Extrakt beispielsweise vor physiologischen Einflüssen, so daß er nicht aus dem Konjugat mit der Gelatine verdrängt wird. Auf diese Weise wird vorteilhaft der Erhalt der molekulardispersen Verteilung des Ginkgo biloba-Extrakts von der Freisetzung aus der Arzneiform bis zur nachfolgenden Resorption sichergestellt. Dieser Schutz kann noch verstärkt werden, indem in die Zubereitung pharmazeutisch übliche Hilfsstoffe, wie beispielsweise Puffersubstanzen (z.B. Dinatriumhydrogenphosphat) eingearbeitet werden.

Desweiteren zeigt eine im physiologischen Milieu schmelzende Ginkgo biloba-Extrakt/Gelatine-Masse hohe Affinität zu Schleimhautoberflächen. Diese Affinität tritt besonders bei Verwendung einer höherbloomigen Gelatine oder fraktionierter Gelatine (Bloomwert oberhalb ca. 200) auf, die sich im physiologischen Milieu nur langsam bzw. verzögert auflöst. An-heften oder Ankleben an der Schleimhaut (Bioadhäsion) bewirkt einen direkten Kontakt des Ginkgo biloba-Extrakts mit der physiologischen Resorptionsbarriere.

Erfindungsgemäße Verwendung von niedriger bloomigen Gelatinesorten (unterhalb ca. 200 Bloom) führt dagegen unter physiologischen Bedingungen zu einer schnelleren Auflösung. Nach dem Schmelzen zeigt hier aber ein Weichmacherzusatz, z.B von Glycerol zur erfindungsgemäßen Zubereitung, die Eigenschaft, eine zähe Masse hoher Viskosität auszubilden, die sich flächenförmig über die Schleimhaut verteilen kann.

Die Anwesenheit von Gelatine bzw. einer von Kollagen abgeleiteten Substanz als Makromolekül primär biogenen Ursprungs stellt darüberhinaus die größtmögliche Verträglichkeit einer erfindungsgemäßen Arzneizubereitung im Organismus sicher. Unerwünschte Nebenwirkungen von Hilfsstoffen, z.B. Irritationen von Schleimhäuten, wie sie etwa durch organische Lösungsmittel oder Tenside hervorgerufen werden können, fallen so ganz weg oder können zumindest entscheidend reduziert werden.

Somit läßt sich zusammenfassend sagen, daß die vorliegende Erfindung eine Zubereitung von Ginkgo biloba-Extrakt zur Verfügung stellt, die dadurch gekennzeichnet ist, daß das Ausmaß der in vivo-Resorption des Ginkgo biloba-Extrakts aus einer molekulardispersen Verteilung in einem hydrophilen Peptid mit einem Molekulargewicht über 100 D im Vergleich zu herkömmlichen Zubereitungen um 50% bis 100% gesteigert wird.

Damit stellt die vorliegende Erfindung einen überaus wertvollen Beitrag zur sinnvollen therapeutischen Nutzung und Anwendung von Ginkgo biloba-Extrakt enthaltenden Phytopharmaka dar.

Unter Ginkgo biloba-Extrakt im Sinne der vorliegenden Erfindung werden sowohl die gesamten Inhaltsstoffe aus Ginkgo biloba, als auch Pflanzenauszüge wie beispielsweise aus frischen, gefrorenen oder getrockneten Blättern, Früchten, Samen, Rinde, Wurzeln, etc. verstanden, als auch solche Extrakte, die von unerwünschten Inhaltsstoffen befreit sind. Ferner soll die Definition des Begriffs Ginkgo biloba-Extrakt aber auch spezielle lipophile oder hydrophile oder durch CO₂-Extraktion gewonnene Pflanzenauszüge, als auch einzelne Bestandteile der Gesamtextrakte umfassen, sowie deren Mischungen.

An einzelnen Inhaltsstoffen sind folgende Substanzen, nach ihrer chemischen Grundstruktur geordnet, zu nennen wie z.B.:
1. Gruppe der Flavonoide und Flavonglykoside, z.B. Kämpferol C₁₅H₁₀O₆, Quercetin C₁₅H₁₀O₇, Isoquercetin, Isorhamnetin, Luteolin, Delphidenon, (+)-Catechin, (-)-Epicatechin, (+)-Gallocatechin, (-)- Galloepicatechin, Kämpferol-3-rhamnoglucosid C₂₇H₃₀O₁₅, Quercetin-3-rhamnoglucosid, Luteolinglykosid, Delphidenonglykosid, Kämpferolglucosid, Quercetin-3-glucosid, Isoquercetinglykosid, 5,7,3',4'-Tetrahydroxy-flavon-3-O-alpharhamnopyranosyl-4''-O-beta-D-(6'''-trans-p-cumaroyl)-glucopyranosid, Cumarsäureester von Quercetin und Kämpferolglucorhamnosid etc.
2. Gruppe der Terpene und Terpenlactone, z.B. Ginkgolid A C₂₀H₂₄O₉, Ginkgolid B C₂₀H₂₄O₁₀, Ginkgolid C C₂₀H₂₄O₁₁, Ginkgolid M C₂₀H₂₄O₁₀, Ginkgolid J C₂₀H₂₄O₁₀, Bilobanon C₁₅H₂₀O₂, Bilobalid A C₁₅H₁₈O₈
3. Gruppe der Biflavonoide, z.B. Ginkgetin C₃₂H₂₂O₁₀ und Isoginkgetin C₃₂H₂₂O₁₀, Bilobetin C₃₁H₂₀O₁₀, Prodelphinidine C₃₀H₂₆O₁₄ oder C₃₀H₂₆O₁₃, Amentoflavon.
4. Steroide, z.B. Sitosterolin C₃₅H₆₀O₆, beta-Sitosterin C₃₅H₆₀O₆, Sitosteringlucosid
5. Carbonsäuren, z.B. Linolensäure, Chinasäure, Shikimisäure, Kynurensäure, Hydroxykynurensäure, p-Hydroxybenzoesäure

Schließlich sollen auch Derivate der obengenannten Verbindungen, wie z.B. Ether, Ester, Komplexe, Salze, Hydrolyseprodukte u.a chemische Molekülvariationen sowie auch synthetische Analoga dieser Stoffe, sowie Mischungen aller vorgenannten Stoffe unter den Extraktbegriff fallen.

Die erfindungsgemäßen Zubereitungen lassen sich nicht nur für pharmazeutische Zwecke als Phytopharmaka einsetzen. Es können darüberhinaus auch kosmetische Anwendungen oder der Einsatz in diätetischen Lebensmitteln möglich sein.

Im folgenden wird das Verfahren zur Herstellung einer erfindungsgemäßen, Flavonoide enthaltenden Zubereitung beschrieben.

Im einfachsten Fall läßt sich das erfindungsgemäße Verfahren zur Herstellung von Flavonoid-Zubereitungen, die wenigstens ein Flavonoid in molekulardisperser Verteilung in einer Basissubstanz aus einem hydrophilen Peptid mit einem Molekulargewicht über 100 D enthalten, mit den folgenden Verfahrensschritten beschreiben:
a) Man überführt das Flavonoid in Lösung und vermischt diese Lösung mit einer wässrigen Lösung der Basissubstanz.
b) Aus dieser Lösung wird das oder werden die Lösungsmittel entfernt.

Die Ausführungsform des erfindungsgemäßen Verfahrens zur Herstellung einer Ginkgo biloba-Extrakt enthaltenden Zubereitung läßt sich im einfachsten Fall mit den folgenden Verfahrensschritten beschreiben:
a) Man suspendiert oder löst den Ginkgo biloba-Extrakt in einem Lösungsmittel und vermischt diese Suspension oder Lösung mit einer wässrigen Lösung eines hydrophilen Peptids mit einem Molekulargewicht über 100 D, ausgewählt aus der Gruppe bestehend aus: Kollagen, Gelatine, fraktionierter Gelatine, Kollagenhydrolysaten, Gelatinederivaten, Pflanzenproteinen, Pflanzenproteinhydrolysaten, Elastinhydrolysaten, Albumine, Caseinhydrolysaten, Casein; sowie deren Mischungen.
b) Aus dieser Lösung werden das oder die Lösungsmittel entfernt.

Üblicherweise beträgt das Mengenverhältnis von Flavonoid zur Basissubstanz 1:0,5 bis 1:1000, insbesondere aber 1:2 bis 1:50, angegeben in Gewichtsteilen Trockensubstanz.

Diesem erfindungsgemäßen System können weitere zusätzliche hydrophile Makromoleküle, die die lösungsvermittelnde Wirkung der Basissubstanz verstärken , u.U. sogar potenzieren können, wie z.B. Agar-Agar, Gummi arabicum, Pektine, Traganth, Xanthan, natürliche sowie modifizierte Stärken, Dextrane, Dextrine, Maltodextrin, Chitosan, Alginate, Cellulosederivate, Polyvinylalkohol, Polyvinylpyrrolidon, Polyethylenglykole, Polyacrylsäure, und Polymere aus Methacrylsäure und Methacrylsäureestern; sowie deren Mischungen, zugesetzt werden.

Das Gewichtsverhältnis dieser hydrophilen Makromoleküle zur Basissubstanz kann bis zu 1:1 betragen.

Dem nach dem beschriebenen erfindungsgemäßen Verfahren hergestellten System können ggf. weitere für die entsprechende Verwendung geeignete Träger- und Hilfsstoffe zugesetzt werden. Solche können z.B. pharmazeutisch übliche Träger- und Hilfsstoffe aus folgenden Gruppen sein:
1. zusätzliche Gerüstbildner,
   z.B. Dextrane, Saccharose, Glycin, Lactose, sowie Mischungen davon.
2. Füllstoffe, z.B. Stärke.
3. Tenside, wie z.B. Polysorbate;
4. pH-Korrigentien, z.B. Dinatriumcitrat, Dinatriumphosphat etc.
5. Farbstoffe, z.B. Curcumin
6. aromatisierende Zusätze bzw. Geschmackskorrigentien, z.B. Fruchtauszüge, Fruchtsaftkonzentrate etc.

In einer Ausführungsform des unter a) beschriebenen Verfahrensschrittes kann das Flavonoid in mit Wasser mischbaren, organischen Lösungsmitteln gelöst werden. Wasser kann bereits zugegen sein, ist aber nicht zwingend notwendig.

Als Lösungsmittel für das Flavonoid, z. B. für den Ginkgo biloba-Extrakt, können beispielsweise mit Wasser mischbare organische Lösungsmittel ausgewählt werden, aus der Gruppe bestehend aus: mit Wasser mischbare organische Lösungsmittelsysteme; niedere Alkohole, wie z.B. Methanol, Ethanol, Isopropanol; niedere Ketone, wie z.B. Aceton; Glycerol, Polyethylenglykole, 1,2-Propylenglykol, Tetrahydrofurfurylalkoholpolyethylenglycolether; niedere Ether; niedere Ester; sowie deren Mischungen.

Je nach lipophilem bzw. hydrophilem Charakter des Extrakts bzw. der Extraktbestandteile kann sich dabei vor der Vermischung mit der wäßrigen Lösung des hydrophilen Peptids eine klare Lösung oder eine Suspension des Ginkgo biloba-Extrakts ergeben.

Die mit diesem einfachen Verfahren erhaltenen, stabilen Lösungen der Flavonoide können bereits als arzneiliche Zubereitungen angesehen und als solche auch verwendet werden.

Die Stabilität des wäßrig/organischen Systems ist, unter in vitro- wie auch in vivo-Bedingungen jedoch nicht an die Anwesenheit des organischen Lösungsvermittlers wie z.B. Alkohol gebunden, wie man zunächst vermuten könnte. Diese Tatsache läßt sich durch Entfernen des Alkohols, z.B. durch Evaporation leicht zeigen, im Gegensatz zu handelsüblichen Arzneizubereitungen (Tropfen) des Standes der Technik.

Dem organischen Zusatz in den erfindungsgemäßen Produkten dürfte damit eine Vehikelfunktion zukommen, indem beispielsweise durch Veränderung der Hydrathülle der von Kollagen abgeleiteten Substanz wie Gelatine deren lipophile Bezirke aktiviert werden. Auf diese Weise können bevorzugt Wechselwirkungen zwischen der Gelatine und lipophilen Molekülteilen von Flavonoiden stattfinden.

Eine Modifizierung des unter a) beschriebenen Verfahrensschrittes ergibt sich im Falle von hydrophilen Peptiden, die sich in kaltem Wasser (Wasser von Raumtemperatur) auflösen, wie z.B. Kollagenhydrolysaten oder Gelatinederivaten. Diese Stoffe können beispielsweise direkt mit einer wäßrig/alkoholischen Ginkgo biloba-Extrakt-Lösung vermischt und somit darin aufgelöst werden.

Eine weitere Ausführungsform des unter a) beschriebenen Verfahrensschrittes zur Herstellung von flüssigen, wäßrigen und stabilen, molekulardispersen Verteilungen von Flavonoiden im Sinne der Erfindung kann dann angewendet werden, wenn ein Flavonglykosid oder sein Aglykon im basischen Medium beispielsweise unter Salzbildung löslich ist. Bei dieser Verfahrensvariante kann auf organische Lösungsmittel mindestens teilweise, aber überraschenderweise auch ganz verzichtet werden.

Üblicherweise werden Flavonoide im Sinne der Erfindung im wäßrig-ammoniakalischen Medium bei pH-Werten oberhalb von 7 gelöst und mit der Basisubstanz, oder wässrigen Lösungen davon homogen vermischt, so daß eine klare Lösung entsteht. Vorteilhaft wird unter Inertgasatmosphäre (z.B. Stickstoff- oder Argonbegasung) gearbeitet, um ein Zersetzen des Flavonoids im basischen Milieu durch Kohlendioxidzutritt zu vermeiden.

Die Salzbildung kann danach unter Säurezusatz rückgängig gemacht werden, oder das Ammoniak durch die im unten beschriebenen Verfahrensschritt der Trocknung (b) angegebenen Methoden, z.B. durch einfache Evaporation oder Gefriertrocknung, entfernt werden.

Die gemäß den bisher angegebenen, erfindungsgemäßen Verfahrensweisen hergestellten molekulardispersen Verteilungen können vor dem nun folgenden Verfahrensschritt b) aufkonzentriert werden oder ein organisches Lösungsmittel kann entfernt werden, z.B. durch Evaporation.

Bei einer weiteren Ausbildungsform der Stufe a) des erfindungsgemäßen Verfahrens wird der Ginkgo biloba-Extrakt zunächst trocken mit dem hydrophilen Peptid mit einem Molekulargewicht über 100 D vermischt und nach Zugabe eines der beschriebenen, flüchtigen, organischen Lösungsmittel (z.B. Alkohol) mit diesem geknetet. Nach dem Trocknen des Lösungsmittels erhält man eine pulverisierbare Masse mit den erfindungsgemäß beschriebenen Eigenschaften.

Bei dem unter b) angegebenen Verfahrensschritt werden das oder die Lösungsmittel aus der in Stufe a) des erfindungsgemäßen Verfahrens erhaltenen Mischung entfernt. Dies kann beispielsweise durch Aufkonzentrierung, Evaporation, Trocknung oder Kombinationen der genannten Prozesse geschehen, wobei sich verschiedene Verfahrensvarianten ergeben können.

### Variante A:

Die Zubereitung wird durch übliche Gefriertrocknung in den trockenen, festen Zustand überführt.

Das Eingefrieren der Zubereitung kann in der Gefriertrocknungsanlage selbst erfolgen, z.B. im Bereich von -10°C bis -40°C. Das vollständige Eingefrieren kann durch eine sprunghafte Änderung der Leitfähigkeit in der zu gefrierenden Probe leicht festgestellt werden. Die eigentliche Trocknung wird bei Temperaturen von 15°C unterhalb des Sublimationspunktes von Wasser bei einem Druck von 0,1 Pa bis 10² Pa (0,001 mbar bis 1,03 mbar) durchgeführt. Der Trocknungsvorgang, der in einer herkömmlichen Gefriertrocknungsanlage (Kondensatortemperatur: ca. -40°C) bei -25°C und 33 Pa (0,33 mbar) in der Primärtrocknung unter Sublimation des gefrorenen Wassers ablaufen kann, führt nach Sekundärtrocknung (Desorption) zu einem gefriergetrockneten Produkt.

### Variante B:

Durch übliche Sprühtrocknung werden Wasser bzw. ein flüchtiges, organisches Lösungsmittel bzw. das Ammoniak entfernt. In herkömmlichen Sprühtrocknungsanlagen verwendet man normalerweise als Prozeßgas Luft mit einer Eintrittstemperatur zwischen 100°C und 300°C, wobei sich eine mittlere Temperaturdifferenz zwischen Prozeßgas und Gut von 50°C bis 100°C ergeben kann. Als Prozeßgas kann bei Vorliegen von besonders oxidationsempfindlichen Substanzen auch ein Inertgas, wie z.B. Stickstoff eingesetzt werden.

Man gewinnt ein trockenes Pulver.

Organische Lösungsmittel können vor der Sprühtrocknung oder Gefriertrocknung entfernt werden, z.B. durch Evaporation und können so einfach und wirtschaftlich zur erneuten Verwendung wiedergewonnen werden.

### Variante C:

In einer Ausführungsform des erfindungsgemäßen Verfahrensschrittes (b) kann beispielsweise durch einfache Lufttrocknung und anschließende Mahlung ein Gelatinegranulat mit erfindungsgemäßen Eigenschaften erhalten werden. Bei der Trocknung kann zur Beschleunigung des Trocknungsvorgangs und zur Einhaltung von niedrigen Temperaturen unter Vakuum (ca. 3000 bis 5000 Pa (ca. 30 bis 50 mbar)), beispielsweise bei Trocknungstemperaturen von bis zu 50°C gearbeitet werden.

Flüssige, erfindungsgemäße Zubereitungen von Ginkgo biloba-Extrakt lassen sich in einer weiteren Ausführungsform des erfindungsgemäßen Verfahrens zu festen oder halbfesten bzw. gelförmigen Formkörpern weiterverarbeiten. Solche Zubereitungen zeichnen sich durch eine stabile Fixierung des molekulardispersen Zustands des Ginkgo biloba-Extrakts in einer Matrix aus dem hydrophilen Peptid, insbesondere von Kollagen abgeleiteten Substanzen aus. Sie lassen sich im wäßrigen bzw. physiologischen Milieu vollständig in einen stabilen, flüssigen, gelösten Zustand zurückführen (redispergieren).

Bevorzugt lassen sich flüssige, Ginkgo biloba-Extrakt enthaltende Zubereitungen direkt, oder nach Aufkonzentrieren, mittels des Cryopel^{R}-Verfahrens (Messer Griesheim GmbH) zu den in zahlreichen Patentanmeldungen der ALFATEC-Pharma GmbH (z. B. P 4201179.5; PCT/DE93/00037) beschriebenen Cryopellets in flüssigem Stickstoff formen und anschließend gefriertrocknen. Auf diese Weise gewinnt man, unter Erhalt der erfindungsgemäßen Eigenschaften, nach einem, für empfindliche Pflanzeninhaltsstoffe wie z.B. Ginkgo biloba-Extrakt geeigneten, schonenden Verfahren gefriergetrocknete Cryopellets, die sich beispielsweise aufgrund ihrer Lagerstabilität, hohen mechanischen Stabilität, guten Fließeigenschaften, variabel gestaltbaren Durchmessers von 0,2 - 12 mm u.a. entweder in konventionelle Hartgelatinekapseln abfüllen lassen oder in Dosierspendern abgefüllt als einzeldosierte Akutformen verwenden lassen.
Durch geeignete Hilfsstoffzusätze, wie z.B. Mannit können mittels Gefriertrocknung extrem schnell in kaltem Wasser auflösbare Zubereitungen ("feste Tropfen") hergestellt werden.

Weist das hydrophile Peptid sol-gel bildende Eigenschaften auf, wie beispielsweise bei Verwendung von Gelatine oder fraktionierter Gelatine mit einem Maximum der Molekulargewichtsverteilung oberhalb von ca. 9,5 x 10⁴ D, lassen sich durch Zusatz der beschriebenen Weichmacher halbfeste bis gelförmige Konsistenzen der Zubereitung einstellen, wie sie auch von konventionellen Weichgelatinekapseln her bekannt sind.

Das Mengenverhältnis des hydrophilen Peptids zu Weichmacher kann von 1:0,001 bis 1:50, insbesondere von 1:0,1 bis 1:5, angegeben in Gewichtsteilen, variiert werden.

Dabei zeigt sich, daß die Weichmachersubstanzen, die chemisch gesehen zur Gruppe der Polyole gehören, neben einem Einfluß auf die Hydrathülle des hydrophilen Peptids, wie z.B. Gelatine oder fraktionierte Gelatine, verbunden mit einer Aktivierung lipophiler Molekülbezirke, auch die Stabilisierung und den Grad helikaler Konformation dieser Moleküle bestimmen können. Dadurch können beispielsweise noch intensivere Wechselwirkungen (Konjugatbildung) zwischen der Gelatine bzw. fraktionierten Gelatine und lipophilen Molekülteilen des Ginkgo biloba-Extraktes stattfinden. Erstaunlicherweise kann somit der Beladungsgrad der erfindungsgemäßen Matrix mit Ginkgo biloba-Extrakt sehr hoch gewählt werden (bis 0,5:1), ohne daß sich Probleme mit der Stabilität der molekulardispersen Verteilung des Extrakts ergeben.

Besonders schonend und zu einem optisch ansprechenden Produkt mit erstaunlicher technologischer Vielfalt und Vorteilen läßt sich eine solche erfindungsgemäße Zubereitung zu Cryopellets formen, die durch direktes Eintropfen bzw. Eindosieren der zu verarbeitenden Masse in ein Tauchbad mit Flüssigstickstoff, z.B. mittels des Cryopel^{R}-Verfahrens (siehe oben) hergestellt werden. Im Bedarfsfall kann durch Trocknung solcher Cryopellets ein Produkt erhalten werden, das sich vorteilhaft in Hartgelatinekapseln abfüllen läßt, optisch sehr ansprechend ist, und auf einfachem und wirtschaftlichem Wege gewonnen wird, unter Erhalt der erfindungsgemäßen Eigenschaften.

Solche halbfesten bis gelförmigen Zubereitungen können auch als Füllgut, sowohl für konventionelle Weichgelatinekapseln dienen, als auch als Füllgut für Weichgelatinekapseln, die nach einem Tropfverfahren durch Eindosieren in Flüssigstickstoff geformt werden.

Halbfeste Massen sind auch in andere geeignete Behältnisse, wie z.B. gekühlte Blister oder ähnliche Hohlformen eindosierbar. Die Blister können anschließend direkt versiegelt werden und man gewinnt einzeldosierbare Arzneiformen.

Neben den bisher bereits erwähnten Ausführungsformen des erfindungsgemäßen Verfahrens ergibt sich eine weitere Ausführungsform aus der Kenntnis über die Qualität des eingesetzten hydrophilen Peptids.

Von Kollagen abgeleiteten Substanzen, z. B. Gelatine ist weiterhin bekannt, daß in Abhängigkeit der Herstellung der betreffenden Gelatine ihr Aschegehalt, d.h. der Entsalzungsgrad, in Abhängigkeit von der Herstellung, variiert. Bei handelsüblichen Gelatinesorten kann der Aschegehalt bis zu 2 Gew.% betragen. Bei guten Qualitäten liegt der Restaschegehalt jedoch herstellungsbedingt bei Werten unterhalb von 0,2 Gew.%.

Für die erfindungsgemäßen Zubereitungen von Flavonoiden eignen sich beispielsweise als Basissubstanzen von Kollagen abgeleitete Substanzen, ausgewählt aus der Gruppe bestehend aus: Gelatine, fraktionierter Gelatine, Kollagenhydrolysaten, Gelatinederivaten; sowie deren Mischungen.

In Abhängigkeit der molekularen Zusammensetzung der verwendeten, von Kollagen abgeleiteten Substanz ergeben sich unterschiedliche Ausbildungsformen des erfindungsgemäßen Verfahrens.

Bei Verwendung von Kollagenhydrolysaten läßt sich der Verfahrensschritt a) ohne Anwendung von Wärme, z.B. bei Raumtemperatur durchführen. In Kombination mit anschließender Gefriertrocknung wird auf diese Weise eine thermische Belastung empfindlicher und thermolabiler Flavonoide während des gesamten Herstellungsprozesses wirksam vermieden.

Im Falle von Gelatine ist Verfahrensschritt a) entsprechend zu modifizieren. Um die Gelatine in eine wässrige Lösung zu überführen, ist Anwendung von Wärme (z.B. 30-45°C) erforderlich.

Die Kenntnis dieses Sachverhaltes läßt sich in einer weiteren Ausführungsform des erfindungsgemäßen Verfahrens ausnutzen:

Von Flavonoiden ist bekannt, daß sie mit Calcium- oder Magnesiumionen Komplexe bilden können. Diese Zusätze von zweiwertigen Ionen bewirken durch Wechselwirkung mit den polaren Gruppen von Flavonoiden eine Konjugat-(Komplexbildung) der beiden Komponenten. Die bevorzugten Koordinationsstellen des Calciums bzw. Magnesiums können dabei eine Hydroxylgruppe am Kohlenstoffatom 3, eine Hydroxylgruppe am Kohlenstoffatom 5 zusammen mit der benachbarten Ketogruppe (Kohlenstoffatom 4) am Flavonoidgrundgerüst darstellen. Die Bildung solcher Komplexe ist visuell durch eine bathochrome Verschiebung der Absorptionsmaxima angezeigt.

Wechselwirkungen solcher Komplexe aus Flavonoiden mit Calcium- oder Magnesiumsalzen mit den funktionellen Gruppen des Gelatinemoleküls führen zu einer wirkungsvollen Stabilisierung im Sinne der Erfindung, die ohne Gelatine nicht möglich wäre und daher zu Ausfällungen des Flavonoids im physiologischen Milieu führen würde.

Daher wird bei dieser Ausführungsform des erfindungsgemäßen Verfahrens nun beispielsweise eine handelsübliche Gelatine mit einem höheren Aschegehalt (Salzgehalt) verwendet. Andererseits kann einer herstellungsbedingt vollentsalzten Gelatinesorte eine definierte Menge von Calcium- oder Magnesiumsalzen (z.B. 1 bis 2 Gew.% Calciumchlorid oder Magnesiumchlorid) kontrolliert zugesetzt werden.

Die erfindungsgemäßen Zubereitungen können sowohl für pharmazeutische, kosmetische, als auch für diätetische (health care) Zwecke geeignet sein.

Pulverförmige Zubereitungen können durch Konfektionierung in geeignete Behältnisse (z.B. Beutel) zur Herstellung von flüssigen, wäßrigen, einnehmbaren Arzneilösungen dienen. Das Pulver kann ebenso in übliche Hartgelatinekapseln abgefüllt werden, zu klassischen Pellets oder Granulaten weiterverarbeitet werden und dann in Hartgelatinekapseln abgefüllt werden. Komprimierung nach üblichen Methoden zu Tabletten, Herstellung von Dragees etc., ggf. unter Anwendung üblicher Tablettierhilfsstoffe, wie z.B. FST-Komplex, ist ebenfalls möglich.

Erfindungsgemäß lyophilisierte Zubereitungen, besonders bei Verwendung niedermolekularer Gelatinesorten, Kollagenhydrolysaten oder Gelatinederivaten lassen sich in kaltem Wasser beschleunigt wiederauflösen. Solche Zubereitungen können als Basis für Parenteralia, wie z.B. Injektionslösungen dienen.

Auch transdermale Zubereitungen bzw. Verarbeitung einer erfindungsgemäßen Zubereitung mit üblichen Hilfsstoffen zu einer transdermalen Zubereitung ist möglich.

Im Falle von lichtempfindlichen Substanzen, wie z.B. Rutin, muß die Verarbeitung prinzipiell unter Lichtschutz erfolgen.

Im Rahmen seines Fachwissens kann der Fachmann leicht selbständig weitere Verfahrensvarianten ausarbeiten. Folgende Beispiele sollen die Erfindung näher erläutern:

### Beispiel 1:

150 g eines Kollagenhydrolysats mit einem Aschegehalt unterhalb 0,2 Gew.% werden bei Raumtemperatur in 5 l destilliertem Wasser gelöst.

30 g Rutin werden in 2 l Ethanol gelöst und mit der Kollagenhydrolysatlösung homogen vermischt, bis eine klare Lösung entsteht.

Durch Sprühtrocknung, bei der die Eintrittstemperatur des Prozeßgases 180°C und die Austrittstemperatur 80°C beträgt, wird das Lösungsmittel entzogen und man erhält ein trockenes Pulver. Als Prozeßgas wird Stickstoff verwendet.

150 mg des Pulvers (entsprechend 25 mg Rutin) werden in 25 ml künstlichem Magensaft bei 37°C wiederaufgelöst. Es ergibt sich eine klare Lösung.

Das erhaltene Pulver wird nach Trockengranulation mit üblichen Tablettierhilfsstoffen (FST-Komplex) gemischt und auf einer Tablettenmaschine zu Tabletten mit einem Gehalt von 25 mg Rutin verpreßt.

### Beispiel 2:

150 g einer fraktionierten Gelatine mit einem Peptidanteil unterhalb von 1% werden in 3 l destilliertes Wasser gegeben und 45 Minuten vorgequollen. Die Gelatine wird anschließend bei 40°C vollständig aufgelöst.

30 g Rutin werden in 2 l Ethanol gelöst und mit der Gelatinelösung vermischt. Die klare Lösung wird dann, wie in Beispiel 1 angegeben, sprühgetrocknet.

Das erhaltene Pulver wird in opake Hartgelatinekapseln mit einem Gehalt von 25 mg Rutin abgefüllt.

### Beispiel 3:

300 g eines handelsüblichen Kollagenhydrolysats mit einem Aschegehalt unterhalb 0,6 Gew.% werden bei Raumtemperatur in 1,5 l destilliertem Wasser gelöst. In dieser Lösung suspendiert man 90 g Rutin. Unter Stickstoffatmosphäre wird solange Ammoniaklösung (25%) unter Rühren zugegeben bis eine klare Lösung entsteht.

Diese Lösung wird in einer Gefriertrocknungsanlage bei -35°C tiefgefroren und durch Primärtrocknung bei -25°C und 5 Pa (0,05 mbar) und einer Sekundärtrocknung bei 20°C getrocknet.

Das erhaltene Produkt löst sich in kaltem Wasser vollständig und klar auf und kann zur Bereitung von peroral einzunehmenden Lösungen verwendet werden.

### Beispiel 4:

300 g eines vollentsalzten Gelatinepulvers (100 Bloom) mit einem Aschegehalt unterhalb 0,2 Gew.% werden in 6 l destilliertes Wasser gegeben und 45 Minuten vorgequollen. Die Gelatine wird anschließend bei 45°C vollständig aufgelöst.

Zu der Gelatinelösung gibt man 6 g Calciumchlorid und 90 g Rutin. Unter Stickstoffatmosphäre wird solange Ammoniakösung (25%) unter Rühren zugegeben bis eine klare Lösung entsteht.

Die Lösung wird anschließend sprühgetrocknet (Eintrittstemperatur 200°C Austrittstemperatur 90°C, Prozeßgas ist Stickstoff).

Man erhält ein intensiv gelb gefärbtes Pulver, das in opake Hartgelatinekapseln mit einem Gehalt von jeweils 40 mg Rutin abgefüllt wird.

### Beispiel 5:

300 g eines handelsüblichen Gelatinepulvers (60 Bloom) mit einem Aschegehalt unterhalb 2 Gew.% werden in 6 l destilliertes Wasser gegeben und 45 Minuten vorgequollen. Die Gelatine wird anschließend bei 40°C vollständig aufgelöst.

Zu der Gelatinelösung gibt man 75 g Rutin. Unter Stickstoffatmosphäre wird solange Ammoniakösung (25%) unter Rühren zugegeben bis eine klare Lösung entsteht.

Diese Lösung wird in einer Gefriertrocknungsanlage bei -30°C tiefgefroren und durch Primärtrocknung bei -20°C und 10 Pa (0,1 mbar) und einer Sekundärtrocknung bei 25°C getrocknet.

250 mg des Pulvers (entsprechend 50 mg Rutin) werden in 25 ml künstlichem Magensaft bei 37°C wiederaufgelöst. Es ergibt sich eine klare, intensiv gelb gefärbte Lösung.

### Beispiel 6:

150 g Gelatinepulver (30 Bloom) mit einem Aschegehalt unterhalb 0,2 Gew.% werden in 5 l dest. Wasser 20 min vorgequollen und danach bei 40°C eine Lösung hergestellt.

40 g Ginkgo biloba-Extrakt werden mit 1 l Ethanol angerührt und mit der Gelatinelösung homogen vermischt, bis eine klare Lösung entsteht.

Durch Sprühtrocknung, bei der die Eintrittstemperatur des Prozessgases bis zu 180°C und die Austrittstemperatur 80°C beträgt, wird Lösungsmittel entzogen und man erhält ein Pulver.
Als Prozessgas wird Stickstoff verwendet.

190 mg des Pulvers (entsprechend 40 mg Ginkgo biloba-Extrakt) werden in 25 ml künstlichem Magensaft bei 37°C wiederaufgelöst. Es ergibt sich eine klare Lösung.

Das erhaltene Pulver wird nach Trockengranulation mit üblichen Tablettierhilfsstoffen (FST-Komplex) gemischt und auf einer Tablettenmaschine zu Tabletten mit einem Gehalt von 40 mg Ginkgo biloba-Extrakt verpreßt.

### Beispiel 7:

Die Durchführung erfolgt analog Beispiel 6 unter Verwendung eines Gelatinepulvers mit 150 Bloom.

Das analog Beispiel 6 erhaltene Pulver wird nach Vermischen mit Lactose als Füllstoff zu Hartgelatinekapseln mit einem Gehalt von 40 mg Ginkgo biloba-Extrakt weiterverarbeitet.

### Beispiel 8:

150 g handelsübliches Kollagenhydrolysat, Aschegehalt unterhalb 2Gew.%, Molekulargewicht 3000 g/mol.
150 g Mannit
700 g dest. Wasser

Das Mannit und das Kollagenhydrolysat werden in 350 g des Wassers gelöst. Durch Zugabe von Ammoniaklösung wird ein pH-Wert zwischen 8 und 10 eingestellt.

50 g Ginkgo biloba-Extrakt werden in 350 g Wasser homogen verteilt und unter Stickstoffbegasung mittels Ammoniaklösung ein pH-Wert zwischen 8 und 10 eingestellt.

Beide Lösungen werden vermischt und durch Eintropfen der Masse in ein Tauchbad mit Flüssigstickstoff mittels des Cryopel^{R}-Dosiersystems werden Pellets mit einem Durchmesser von 5 mm geformt.

Die gefrorenen Pellets werden in einer Gefriertrocknungsanlage mit einer Primärtrocknung bei -25°C und 5 Pa (0,05 mbar) und einer Sekundärtrocknung bei 22°C getrocknet.

Die getrockneten Pellets können als einzeldosierbare Arzneiform in einen Dosierspender abgefüllt werden. Sie sind in kaltem Wasser leicht löslich, wobei sich eine klare Lösung des Ginkgo biloba-Extrakts ergibt.

### Beispiel 9:

1000 g Gelatinepulver 250 Bloom, Aschegehalt unterhalb 0,2 Gew.%
750 g Glycerin 85%
3250 g dest. Wasser

Das Gelatinepulver wird nach 30 min vorquellen im Wasser bei 50°C vollständig aufgelöst.

300 g Ginkgo biloba-Extrakt werden in dem Glycerin und 500 ml Ethanol bei 50°C gelöst und danach homogen mit der Gelatinelösung vermischt.

Die klare Masse wird mittels einer Cryopel^{R}-Dosiervorrichtung durch Eintropfen in ein Tauchbad mit Flüssigstickstoff zu Pellets geformt.

Die Pellets werden auf herkömmliche Weise bei Temperaturen zwischen 20°C bis 40°C getrocknet und anschließend in opake Hartgelatinekapseln abgefüllt, mit einem Gehalt von 30 mg Ginkgo biloba-Extrakt je Hartgelatinekapsel.

Der Inhalt einer Hartgelatinekapsel löst sich in künstlichem Magensaft (pH1) bei 37°C innerhalb von 30 min vollständig auf, wobei eine klare Lösung des Ginkgo biloba-Extrakts entsteht.

### Beispiel 10:

Klinische Studien zur Bioverfügbarkeit von Rutin wurden unter den folgenden Bedingungen durchgeführt:

An einer Gruppe von 4 gesunden Probanden wurde über 24 Stunden die renale Ausscheidung von Quercetin, Kämpferol und Isorhamnetin nach Verabreichung von 600 mg Rutin als Zubereitung mit Gelatine (im Vergleich zu einem handelsüblichen Rutin-Fertigarzneimittel (als Kontrolle) gleicher Dosierung) gemessen. Die nahrungsbedingte Flavonausscheidung wurde dabei berücksichtigt und durch entsprechende Diätvorschriften möglichst gering gehalten.

Die Analytik und Identifikation der Flavonausscheidungen kann z.B. wie folgt durchgeführt werden:
1. Saure Spaltung ( 1 M Salzsäure) der in biologischen Flüssigkeiten (Urin) enthaltenen Flavonkonjugate.
2. Festphasenextraktion mit Methanol zur Anreicherung der freigesetzten Flavone (z.B. Extrelut^{R}, Fa. Merck).
3. HPLC-Analytik mit Diodenarray-Detektion und Identifizierung einzelner Flavone, zusätzlich elektrochemische Identifikation (anodisches Oxidationspotential).

Die Empfindlichkeit der genannten Analytik ist >10 ng Flavon pro ml.

Die renalen Flavonausscheidungen betrugen:

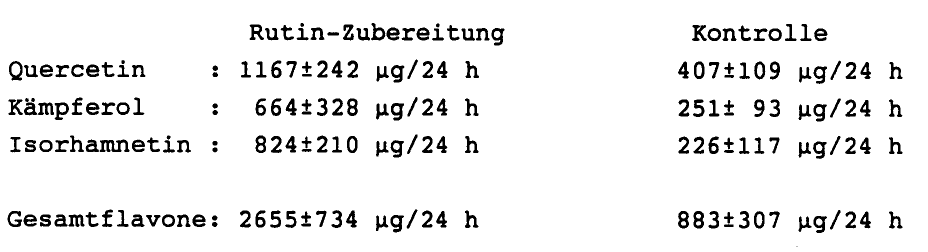

Bezogen auf das Ausscheidungsprofil von Quercetin entspricht die Nettoausscheidung an Gesamtflavonen einer Resorptionsquote von 5,9±2,3 %.

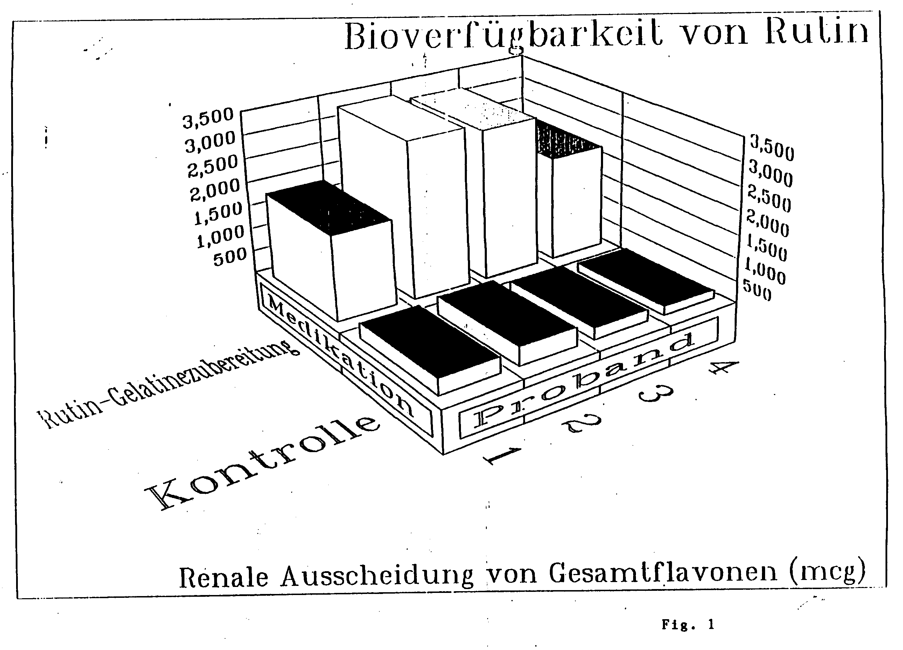

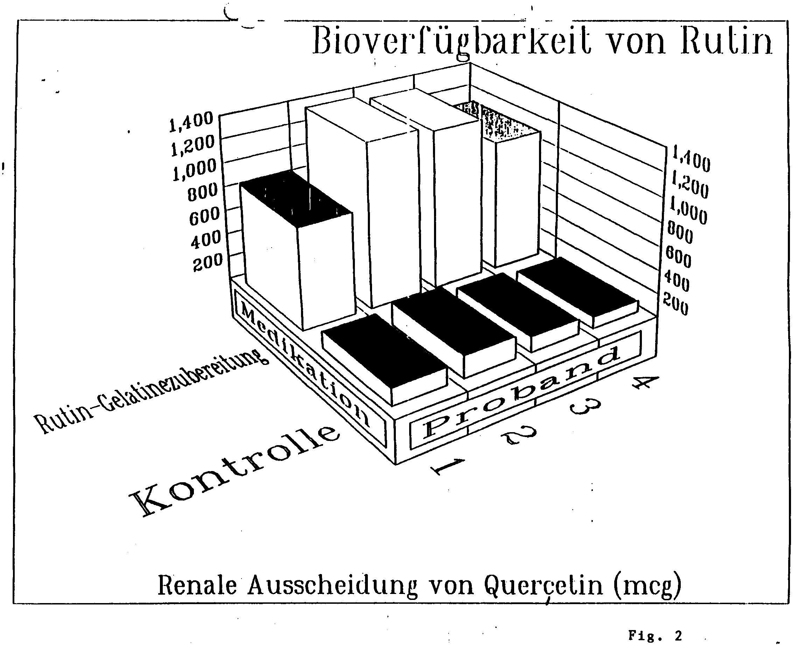

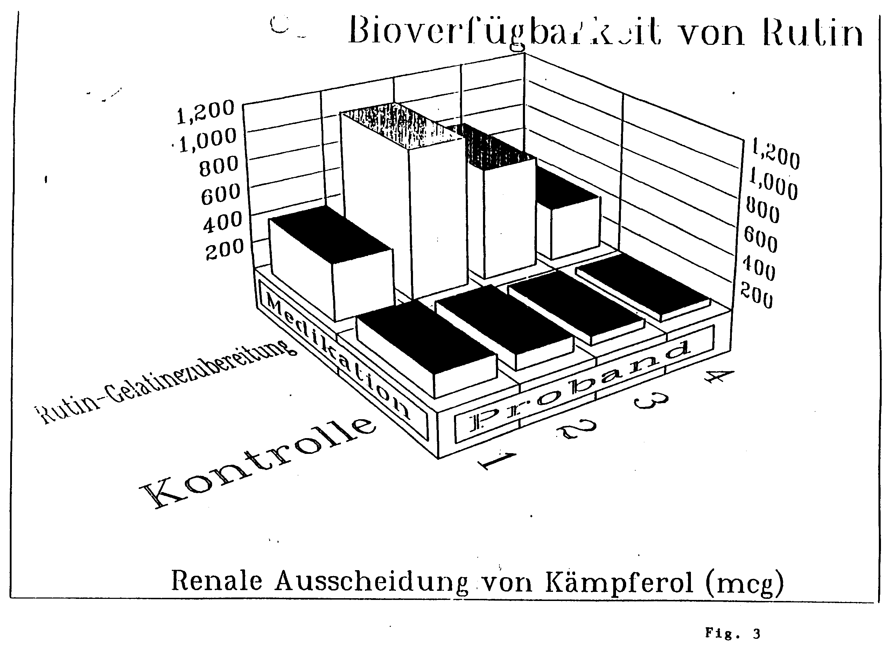

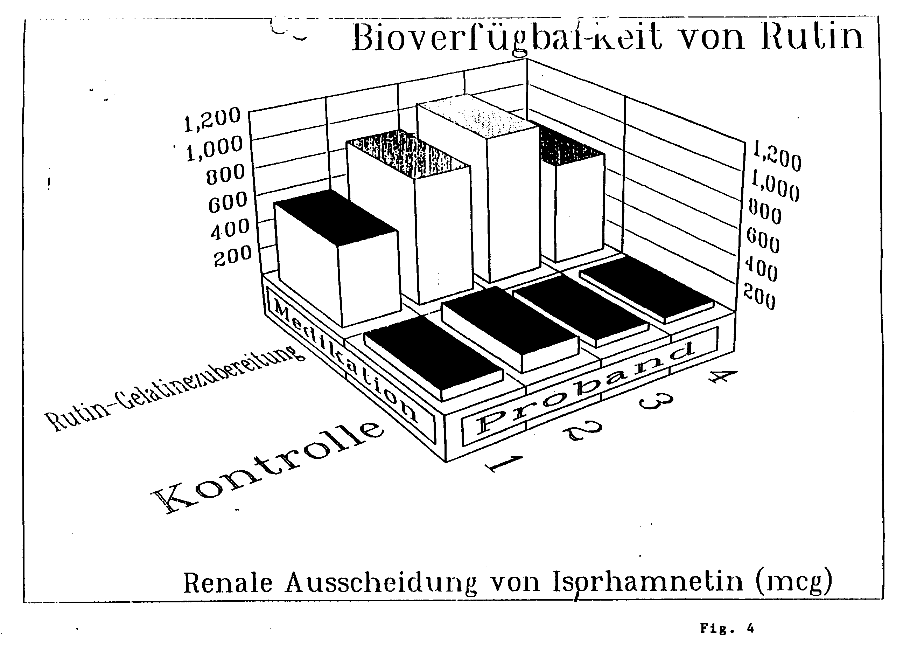

Die erhaltenen Werte sind in den Figuren 1 bis 4 dargestellt. Dabei zeigen:
Fig.1 die renale Ausscheidung von Gesamtflavonen
Fig.2 die renale Ausscheidung von Quercetin
Fig.3 die renale Ausscheidung von Kämpferol
Fig.4 die renale Ausscheidung von Isorhamnetin.

Wie man den Figuren entnimmt, wurde nicht nur der bekannte Rutin-Metabolit Quercetin, sondern auch die als Metaboliten des Rutins in vivo erstmals nachgewiesenen Verbindungen Kämpferol und Isorhamnetin qualitativ und quantitativ in den renalen Ausscheidungen nachgewiesen.

Damit wurde ein Metabolitspektrum des Rutins erhalten, das im wesentlichen dem Wirkstoffspektrum des Gingko biloba-Extrakts entspricht.

## Patentansprüche

1. Flavonoid-Zubereitung, enthaltend wenigstens ein Flavonoid in molekulardisperser Verteilung in einer Basissubstanz aus einem hydrophilen Peptid mit einem Molekulargewicht zwischen 100 D und 10⁸ D.

2. Flavonoid-Zubereitung nach Anspruch 1, dadurch gekennzeichnet, daß das hydrophile Peptid Kollagen, eine von Kollagen abgeleitete Substanz oder eine Mischung aus von Kollagen abgeleiteten Substanzen ist.

3. Flavonoid-Zubereitung nach Anspruch 1 und/oder 2, dadurch gekennzeichnet, daß das Peptid ein Sol-Gel-Bildner ist.

4. Flavonoid-Zubereitung nach einem der Ansprüche 1 - 3, dadurch gekennzeichnet, daß das Peptid Gelatine, fraktionierte Gelatine, ein Kollagenhydrolysat oder ein Gelatinederivat ist.

5. Zubereitung nach Anspruch 3, dadurch gekennzeichnet, daß die Gelatine oder fraktionierte Gelatine ein Maximum der Molekulargewichtsverteilung oberhalb von 9,5 x 10⁴ D besitzt.

6. Zubereitung nach Anspruch 1, dadurch gekennzeichnet, daß das Peptid ein Elastinhydrolysat ist.

7. Zubereitung nach Anspruch 1, dadurch gekennzeichnet, daß das Peptid ein Pflanzenprotein oder ein Pflanzenproteinhydrolysat ist.

8. Flavonoid-Zubereitung nach einem der Ansprüche 1 bis 7, dadurch gekennzeichnet, daß sie in flüssiger Form vorliegt.

9. Flavonoid-Zubereitung nach einem der Ansprüche 1 bis 7, dadurch gekennzeichnet, daß sie in fester Form vorliegt.

10. Zubereitung nach einem der Ansprüche 1 bis 9, dadurch gekennzeichnet, daß das Gewichtsverhältnis von Flavonoid zu dem hydrophilen Peptid 1:0,5 bis 1:1000 beträgt.

11. Zubereitung nach einem der Ansprüche 1 bis 10, enthaltend zusätzliche hydrophile Makromoleküle aus der Gruppe: Agar-Agar, Gummi arabicum, Pektine, Traganth, Xanthan, natürliche sowie modifizierte Stärken, Dextrane, Dextrine, Maltodextrin, Chitosan, Alginate, Cellulosederivate, Polyvinylalkohol, Polyvinylpyrrolidon, Polyethylenglykole, Polyacrylsäure, und Polymere aus Methacrylsäure und Methacrylsäureestern; sowie deren Mischungen, in einem Gewichtsverhältnis zu dem hydrophilen Peptid bis zu 1:1.

12. Zubereitung nach einem der Ansprüche 1 bis 11, dadurch gekennzeichnet, daß sie einen oder mehrere Weichmacher aus der Gruppe: Glycerol, Propylenglykol, Polyethylenglykole, Triacetin, Sorbitol, Sorbitangemische, Sorbitollösungen, Glucosesirup, Polyole, Zuckeralkohole; sowie deren Mischungen, enthält.

13. Zubereitung nach Anspruch 12, dadurch gekennzeichnet, daß das Gewichtsverhältnis von Peptid zu Weichmacher 1:0,001 bis 1:50 beträgt.

14. Zubereitung nach einem der Ansprüche 1 - 13, dadurch gekennzeichnet, daß das Flavonoid ein Gingko-biloba-Extrakt ist.

15. Zubereitung nach einem der Ansprüche 1 -13, dadurch gekennzeichnet, daß das Flavonoid Rutin ist.

16. Verfahren zur Herstellung einer Flavonoid-Zubereitung, die wenigstens ein Flavonoid in molekulardisperser Verteilung enthält, dadurch gekennzeichnet, daß man
a) das Flavonoid in eine Suspension oder Lösung überführt und diese Suspension oder Lösung mit einer wäßrigen Lösung eines hydrophilen Peptids mit einem Molekulargewicht zwischen 100 D und 10⁸ D aus der Gruppe: Kollagen, Gelatine, fraktionierte Gelatine, Kollagenhydrolysate, Gelatinederivate, Pflanzenproteine, Pflanzenproteinhydrolysate, Elastinhydrolysate von Kollagen abgeleitete Substanzen; sowie deren Mischungen, vermischt, und
b) das oder die Lösungsmittel entfernt.

17. Verfahren nach Anspruch 16, dadurch gekennzeichnet, daß man als hydrophiles Peptid Gelatine oder fraktionierte Gelatine mit einem Maximum der Molekulargewichtsverteilung oberhalb 9,5x10⁴ D einsetzt.

18. Verfahren nach Anspruch 16 und/oder 17, dadurch gekennzeichnet, daß man in Stufe a) das Flavonoid in einem mit Wasser mischbaren organischen Lösungsmittel, ausgewählt aus der Gruppe: mit Wasser mischbare organische Lösungsmittelsysteme; niedere Alkohole wie Methanol, Ethanol, Isopropanol; niedere Ketone wie Aceton; Glycerol, Polyethylenglykole, 1,2-Propylenglykol, Tetrahydrofurfurylalkoholpolyethylenglycolether; niedere Ether; niedere Ester; sowie deren Mischungen löst.

19. Verfahren nach einem der Ansprüche 16 bis 18, dadurch gekennzeichnet, daß man in Stufe a) das Flavonoid unter Salzbildung löst.

20. Verfahren nach Anspruch 19, dadurch gekennzeichnet, daß man im Anschluß an Stufe a) die Salzbildung wieder rückgängig macht.

21. Verfahren nach Anspruch 19, dadurch gekennzeichnet, daß man die Salzbildung mittels Ammoniak durchführt.

22. Verfahren nach Anspruch 16 und/oder 17, dadurch gekennzeichnet, daß man ein flüchtiges, mit Wasser mischbares, organisches Lösungsmittel in Stufe a) einsetzt und dieses vor oder in Stufe b) entfernt.

23. Verfahren nach einem der Ansprüche 16 bis 22, dadurch gekennzeichnet, daß man die Mischung aus Flavonoid und hydrophilem Peptid in Stufe b) trocknet.

24. Verfahren nach Anspruch 23, dadurch gekennzeichnet, daß man vor dem Trocknen die Mischung aus Ginkgo biloba-Extrakt und hydrophilem Peptid zu Cryopellets formt.

25. Verfahren nach einem der Ansprüche 16 bis 24, dadurch gekennzeichnet daß man in Stufe a) zusätzliche hydrophile Makromoleküle aus der Gruppe: Agar-Agar, Gummi arabicum, Pektine, Traganth, Xanthan, natürliche sowie modifizierte Stärken, Dextrane, Dextrine, Maltodextrin, Chitosan, Alginate, Cellulosederivate, Polyvinylalkohol, Polyvinylpyrrolidon, Polyethylenglykole, Polyacrylsäure, und Polymere aus Methacrylsäure und Methacrylsäureestern; sowie deren Mischungen, in einem Gewichtsverhältnis zu dem hydrophilen Peptid bis zu 1:1 zusetzt.

26. Verfahren nach einem der Ansprüche 16 bis 25, dadurch gekennzeichnet, daß man der Mischung aus Flavonoid und hydrophilem Peptid einen oder mehrere Weichmacher aus der Gruppe: Glycerol, Propylenglykol, Polyethylenglykole, Triacetin, Sorbitol, Sorbitangemische, Sorbitollösungen, Glucosesirup, Polyole, Zuckeralkohole; sowie deren Mischungen, zusetzt.

27. Verfahren nach einem der Ansprüche 16 bis 26, dadurch gekennzeichnet, daß man aus der Mischung aus Flavonoid, hydrophilem Peptid und Weichmacher durch Eintropfen in flüssigen Stickstoff Weichgelatinekapseln formt.

28. Verfahren nach einem der Ansprüche 16 bis 26, dadurch gekennzeichnet, daß man aus der Mischung aus Flavonoid und hydrophilem Peptid mit Weichmacher durch Eintropfen in flüssigen Stickstoff Cryopellets formt.

29. Verfahren nach einem der Ansprüche 16 bis 28, dadurch gekennzeichnet, daß man ein hydrophiles Peptid mit einem Aschegehalt unterhalb von 2 Gew.%, vorzugsweise unterhalb von 0,2 Gew.%, einsetzt.

30. Verfahren nach Anspruch 29, dadurch gekennzeichnet, daß man dem hydrophilen Peptid kontrolliert Calcium- oder Magnesiumsalz zusetzt.

31. Verfahren nach einem der Ansprüche 16 bis 30, dadurch gekennzeichnet, daß man in Stufe a) oder nach Stufe b) pharmazeutisch übliche Träger- und Hilfsstoffe zusetzt.

32. Verfahren zur Herstellung einer ein Flavonoid enthaltenden Zubereitung, dadurch gekennzeichnet, daß man das Flavonoid und ein hydrophiles Peptid mit einem Molekulargewicht zwischen 100 D und 10⁸ D trocken mischt und mit einem flüchtigen organischen Lösungsmittel aus der Gruppe: Methanol, Ethanol, Isopropanol, Aceton; sowie deren Mischungen knetet und anschließend trocknet.

33. Verfahren nach einem der Ansprüche 16 - 32, dadurch gekennzeichnet, daß man als Flavonoid einen Gingko biloba-Extrakt einsetzt.

34. Verfahren nach einem der Ansprüche 16 - 32, dadurch gekennzeichnet, daß man als Flavonoid Rutin einsetzt.

35. Verwendung einer Flavonoid-Zubereitung nach einem der Ansprüche 1 bis 15 zur Herstellung einer pharmazeutischen Zubereitung.

36. Verwendung einer Flavonoid-Zubereitung nach einem der Ansprüche 1 bis 15 zur Herstellung einer kosmetischen Zubereitung.

37. Verwendung einer Flavonoid-Zubereitung nach einem der Ansprüche 1 bis 15 zur Herstellung eines diätetischen Lebensmittels (health care).

38. Verwendung einer Flavonoid-Zubereitung gemäß einem der Ansprüche 1 bis 15 enthaltend Rutin als Flavonoid zur Herstellung eines Arzneimittels zur Erzielung der pharmakologischen Wirkung von Quercetin.

39. Verwendung einer Flavonoid-Zubereitung gemäß einem der Ansprüche 1 bis 15 enthaltend Rutin als Flavonoid zur Herstellung eines Arzneimittels zur Erzielung der pharmakologischen Wirkung von Kämpferol.

40. Verwendung einer Flavonoid-Zubereitung gemäß einem der Ansprüche 1 bis 15 enthaltend Rutin als Flavonoid zur Herstellung eines Arzneimittels zur Erzielung der pharmakologischen Wirkung von Isorhamnetin.

41. Verwendung einer Flavonoid-Zubereitung gemäß einem der Ansprüche 1 bis 15 enthaltend Rutin als Flavonoid zur Herstellung eines Arzneimittels zur Erzielung der pharmakologischen Wirkung von einer Mischung aus Quercetin, Kämpferol und Isorhamnetin.

42. Verwendung einer Flavonoid-Zubereitung gemäß einem der Ansprüche 1 bis 15 enthaltend Rutin als Flavonoid zur Herstellung eines Arzneimittels zur Erzielung der wichtigsten pharmakologischen Wirkungen des Gingko biolaba-Extraktes.

## Claims

1. A flavonoid preparation, containing at least one flavonoid in molecular disperse distribution in a base substance of a hydrophilic peptide having a molecular weight of between 100 D and 10⁸ D.

2. A flavonoid preparation according to claim 1, characterised in that said hydrophilic peptide is collagen, a substance derived from collagen, or a mixture of substances derived from collagen.

3. A flavonoid preparation according to claim 1 and/or 2, characterised in that said peptide is a sol/gel forming agent.

4. A flavonoid preparation according to any one of claims 1 to 3, characterised in that said peptide is gelatin, fractionated gelatin, a collagen hydrolysate, or a gelatin derivative.

5. A preparation according to claim 3, characterised in that the gelatin or fractionated gelatin has a maximum of molecular weight distribution above 9.5 × 10⁴ D.

6. A preparation according to claim 1, characterised in that said peptide is an elastin hydrolysate.

7. A preparation according to claim 1, characterised in that said peptide is a plant protein or a plant protein hydrolysate.

8. A flavonoid preparation according to any one of claims 1 to 7, characterised in that it is present in liquid form.

9. A flavonoid preparation according to any one of claims 1 to 7, characterised in that it is present in solid form.

10. A preparation according to any one of claims 1 to 9, characterised in that the weight ratio of flavonoid to said hydrophilic peptide is 1:0.5 to 1:1000.

11. A preparation according to any one of claims 1 to 10, containing additional hydrophilic macromolecules from the group: agar-agar, gum arabic, pectins, tragacanth gum, xanthene, natural and modified starches, dextranes, dextrines, maltodextrin, chitosan, alginates, cellulose derivatives, polyvinyl alcohol, polyvinyl pyrrolidone, polyethylene glycols, polyacrylic acid, and polymers of methacrylic acid and methacrylic acid esters; as well as mixtures thereof, in a weight ratio to said hydrophilic peptide of up to 1:1.

12. A preparation according to any one of claims 1 to 11, characterised in that it contains one or more softening agents from the group: glycerol, propylene glycol, polyethylene glycols, triacetin, sorbitol, sorbitan mixtures, sorbitol solutions, glucose syrup, polyols, sugar alcohols; as well as mixtures thereof.

13. A preparation according to claim 12, characterised in that the weight ratio of peptide to softening agent is 1:0.001 to 1:50.

14. A preparation according to any one of claims 1 to 13, characterised in that said flavonoid is a *Ginkgo biloba* extract.

15. A preparation according to any one of claims 1 to 13, characterised in that said flavonoid is rutin.

16. A method for producing a flavonoid preparation containing at least one flavonoid in molecular disperse distribution, characterised in that
a) said flavonoid is transformed into a suspension or solution, and this suspension or solution is mixed with an aqueous solution of a hydrophilic peptide having a molecular weight of between 100 D and 10⁸ D from the group: collagen, gelatin, fractionated gelatin, collagen hydrolysates, gelatin derivatives, plant proteins, plant protein hydrolysates, elastin hydrolysates, substances derived from collagen; as well as mixtures thereof, and
b) the solvent(s) is (are) removed.

17. A method according to claim 16, characterised in that gelatin or fractionated gelatin having a maximum of molecular weight distribution above 9.5 ×10⁴ D is used as a hydrophilic peptide.

18. A method according to claim 16 and/or 17, characterised in that in step a) said flavonoid is dissolved in an organic solvent miscible with water, selected from the group: organic solvent systems miscible with water; low alcohols such as methanol, ethanol, isopropanol; low ketones such as acetone; glycerol, polyethylene glycols, 1,2-propyleneglycol, tetrahydrofurfurylalcohol polyethyleneglycolether; low ethers; low esters; as well as mixtures thereof.

19. A method according to any one of claims 16 to 18, characterised in that in step a) said flavonoid is dissolved unter salt formation.

20. A method according to claim 19, characterised in that salt formation is neutralised again subsequent to step a).

21. A method according to claim 19, characterised in that salt formation is carried out by means of ammonia.

22. A method according to claim 16 and/or 17, characterised in that a volatile organic solvent miscible with water is used in step a) and removed prior to, or in, step b).

23. A method according to any one of claims 16 to 22, characterised in that said mixture of flavonoid and hydrophilic peptide is dried in step b).

24. A method according to claim 23, characterised in that said mixture of *Ginkgo biloba* extract and hydrophilic peptide is formed into cryopellets prior to drying.

25. A method according to any one of claims 16 to 24, characterised in that in step a) additional hydrophilic macromolecules from the group: agar-agar, gum arabic, pectins, tragacanth gum, xanthene, natural and modified starches, dextranes, dextrines, maltodextrin, chitosan, alginates, cellulose derivatives, polyvinyl alcohol, polyvinyl pyrrolidone, polyethylene glycols, polyacrylic acid, and polymers of methacrylic acid and methacrylic acid esters; as well as mixtures thereof are added to said hydrophilic peptide in a weight ratio of 1:1.

26. A method according to any one of claims 16 to 25, characterised in that one or more softening agents from the group: glycerol, propylene glycol, polyethylene glycols, triacetin, sorbitol, sorbitan mixtures, sorbitol solutions, glucose syrup, polyols, sugar alcohols; as well as mixtures thereof, are added to said mixture of flavonoid and hydrophilic peptide.

27. A method according to any one of claims 16 to 26, characterised in that soft gelatin capsules are formed of said mixture of flavonoid, hydrophilic peptide and softening agent by dropwise introduction into liquid nitrogen.

28. A method according to any one of claims 16 to 26, characterised in that cryopellets are formed of said mixture of flavonoid and hydrophilic peptide with softening agents by dropwise introduction into liquid nitrogen.

29. A method according to any one of claims 16 to 28, characterised in that a hydrophilic peptide having an ash content of less than 2% (wt.), preferably less than 0.2% (wt.) is used.

30. A method according to claim 29, characterised in that calcium salt or magnesium salt is added to said hydrophilic peptide in a controlled manner.

31. A method according to any one of claims 16 to 30, characterised in that pharmaceutically customary carriers and adjuvants are added in step a) or after step b).

32. A method for producing a preparation containing a flavonoid, characterised in that said flavonoid and a hydrophilic peptide having a molecular weight of between 100 D and 10⁸ D are mixed dry and kneaded with a volatile organic solvent from the group: methanol, ethanol, isopropanol, acetone; as well as mixtures thereof, and subsequently dried.

33. A method according to any one of claims 16 to 32, characterised in that a *Ginkgo biloba* extract is used as a flavonoid.

34. A method according to any one of claims 16 to 32, characterised in that rutin is used as a flavonoid.

35. Use of a flavonoid preparation according to any one of claims 1 to 15 for producing a pharmaceutical preparation.

36. Use of a flavonoid preparation according to any one of claims 1 to 15 for producing a cosmetic preparation.

37. Use of a flavonoid preparation according to any one of claims 1 to 15 for producing a dietetic food (health care).

38. Use of a flavonoid preparation according to any one of claims 1 to 15 containing rutin as a flavonoid for producing a drug for obtaining the pharmacological effect of quercetin.

39. Use of a flavonoid preparation according to any one of claims 1 to 15 containing rutin as a flavonoid for producing a drug for obtaining the pharmacological effect of kaempferol.

40. Use of a flavonoid preparation according to any one of claims 1 to 15 containing rutin as a flavonoid for producing a drug for obtaining the pharmacological effect of isorhamnetin.

41. Use of a flavonoid preparation according to any one of claims 1 to 15 containing rutin as a flavonoid for producing a drug for obtaining the pharmacological effect of a mixture of quercetin, kaempferol and isorhamnetin.

42. Use of a flavonoid preparation according to any one of claims 1 to 15 containing rutin as a flavonoid for producing a drug for obtaining the primary pharmacological effects of said *Ginkgo biloba* extract.

## Revendications

1. Préparation de flavonoïde, contenant au moins un flavonoïde à répartition moléculaire dispersée dans une substance de base formée d'un peptide hydrophile de poids moléculaire compris entre 100 D et 10⁸ D.

2. Préparation de flavonoïde suivant la revendication 1, caracterisée en ce que le peptide hydrophile est le collagène, une substance dérivée du collagène ou un mélange de substances dérivées du collagène.

3. Préparation de flavonoïde suivant les revendications 1 et/ou 2, caractérisée en ce que le peptide est un générateur de sol-gel.

4. Préparation de flavonoïde suivant l'une des revendications 1 à 3, caractérisée en ce que le peptide est la gélatine, une gélatine fractionnée, un hydrolysat de collagène ou un dérivé de gélatine.

5. Préparation suivant la revendication 3, caractérisée en ce que la gélatine ou la gélatine fractionnée a un maximum de répartition de poids moléculaire au-dessus de 9,5 x 10⁴ D.

6. Préparation suivant la revendication 1, caractérisée en ce que le peptide est un hydrolysat d'élastine.

7. Préparation suivant la revendication 1, caractérisée en ce que le peptide est une protéine végétale ou un hydrolysat de protéine végétale.

8. Préparation de flavonoïde suivant l'une des revendications 1 à 7, caractérisée en ce qu'elle est sous la forme liquide.

9. Préparation de flavonoïde suivant l'une des revendications 1 à 7, caractérisée en ce qu'elle est sous la forme solide.

10. Préparation suivant l'une des revendications 1 à 9, caractérisée en ce que le rapport en poids du flavonoïde au peptide hydrophile est de 1:0,5 à 1:1000.

11. Préparation suivant l'une des revendications 1 à 10, contenant des macromolécules hydrophiles additionnelles du groupe : agar-agar, gomme arabique, pectines, gomme adragante, gamme xanthane, amidons naturels ainsi que modifiés, dextranes, dextrines, maltodextrine, chitosane, alginates, dérivés de cellulose, polymère d'alcool vinylique, polyvinylpyrrolidone, polyéthylèneglycols, polymère d'acide acrylique et polymères d'acide méthacrylique et d'esters d'acide méthacrylique ; ainsi que des mélanges de ces macromolécules, dans un rapport en poids avec le peptide hydrophile jusqu'à 1:1.

12. Préparation suivant l'une des revendications 1 à 11, caractérisée en ce qu'elle contient un ou plusieurs plastifiants du groupe glycérol, propylèneglycol, polyéthylèneglycols, triacétine, sorbitol, mélanges de sorbitanne, solutions de sorbitol, sirop de glucose, polyols, sucre-alcools ; ainsi que des mélanges de ces plastifiants.

13. Préparation suivant la revendication 12, caractérisée en ce que le rapport en poids du peptide au plastifiant est de 1:0,001 à 1:50.

14. Préparation suivant l'une des revendications 1 à 13, caractérisée en ce que le flavonoïde est un extrait de Gingko biloba.

15. Préparation suivant l'une des revendications 1 à 13, caractérisée en ce que le flavonoïde est la rutine.

16. Procédé de production d'une préparation de flavonoïde, qui contient au moins un flavonoïde de répartition moléculaire dispersée, caractérisé en ce que :
a) on fait passer le flavonoïde en suspension ou en solution et on mélange cette suspension ou solution avec une solution aqueuse d'un peptide hydrophile de poids moléculaire compris entre 100 D et 10⁸ D du groupe : collagène, gélatine, gélatine fractionnée, hydrolysats de collagène, dérivés de gélatine, protéines végétales, hydrolysats de protéines végétales, hydrolysats d'élastine, substances dérivées de collagène ; ainsi que des mélanges de ces peptides, et
b) on élimine le ou les solvants.

17. Procédé suivant la revendication 16, caractérisé en ce qu'on utilise comme peptide hydrophile de la gélatine ou de la gélatine fractionnée ayant un maximum de répartition de poids moléculaire au-dessus de 9,5 x 10⁴ D.

18. Procédé suivant les revendications 16 et/ou 17, caractérisé en ce que dans l'étape a), on dissout le flavonoïde dans un solvant organique miscible à l'eau, choisi dans le groupe : systèmes de solvants organiques miscibles à l'eau ; alcools inférieurs tels que méthanol, éthanol, isopropanol ; cétones inférieures telles qu'acétone ; glycérol, polyéthylèneglycols, 1,2-propylèneglycol, éthers d'alcool tétrahydrofurfurylique de polyéthylèneglycols ; éthers inférieurs ; ainsi que des mélanges de ces solvants

19. Procédé suivant l'une des revendications 16 à 18, caractérisé en ce que dans l'étape a), on dissout le flavonoïde par formation de sel.

20. Procédé suivant la revendication 19, caractérisé en ce qu'à la suite de l'étape a), on fait rétrograder la formation de sel.

21. Procédé suivant la revendication 19, caractérisé en ce qu'on conduit la formation de sel au moyen d'ammoniac.

22. Procédé suivant les revendications 16 et/ou 17, caractérisé en ce qu'on utilise dans l'étape a) un solvant organique volatil miscible à l'eau, et on l'élimine avant ou dans l'étape b).

23. Procédé suivant l'une des revendications 16 à 22, caractérisé en ce qu'on sèche le mélange de flavonoïde et de peptide hydrophile dans l'étape b).

24. Procédé suivant la revendication 23, caractérisé en ce qu'avant le séchage, on transforme le mélange d'extrait de Gingko biloba et de peptide hydrophile en cryopellets.

25. Procédé suivant l'une des revendications 16 à 24, caractérisé en ce que dans l'étape a), on ajoute en supplément des macromolécules hydrophiles du groupe : agar-agar, gomme arabique, pectines, gomme adragante, gomme xanthane, amidons naturels ainsi que modifiés, dextranes, dextrines, maltodextrine, chitosane, alginates, dérivés de cellulose, polymère d'alcool vinylique, polyvinylpyrrolidone, polyéthylèneglycols, polymère d'acide acrylique, et polymères d'acide méthacrylique et d'esters d'acide méthacrylique ; ainsi que des mélanges de ces macromolécules, dans un rapport en poids avec le peptide hydrophile jusqu'à 1:1.

26. Procédé suivant l'une des revendications 16 à 25, caractérisé en ce qu'on ajoute au mélange de flavonoïde et de peptide hydrophile un ou plusieurs plastifiants du groupe : glycérol, propylèneglycol, polyéthylèneglycols, triacétine, sorbitol, mélanges de sorbitanne, solutions de sorbitol, sirop de glucose, polyols, sucre-alcools ; ainsi que des mélanges de ces plastifiants.

27. Procédé suivant l'une quelconque des revendications 16 à 26, caractérisé en ce qu'on forme des capsules molles de gélatine à partir du mélange de flavonoïde, de peptide hydrophile et de plastifiant en faisant tomber ce mélange en gouttes dans de l'azote liquide.

28. Procédé suivant l'une des revendications 16 à 26, caractérisé en ce qu'on forme des cryopellets à partir du mélange de flavonoïde et de peptide hydrophile avec le plastifiant en faisant tomber ce mélange en gouttes dans de l'azote liquide.

29. Procédé suivant l'une des revendications 16 à 28, caractérisé en ce qu'on utilise un peptide hydrophile ayant une teneur en cendres inférieure à 2 % en poids, de préférence inférieure à 0,2 % en poids.

30. Procédé suivant la revendication 29, caractérisé en ce qu'on ajoute de façon contrôlée un sel de calcium ou de magnésium au peptide hydrophile.

31. Procédé suivant l'une des revendications 16 à 30, caractérisé en ce que dans l'étape a) ou après l'étape b), on ajoute des excipients et des substances auxiliaires d'emploi classique en pharmacie.

32. Procédé de production d'une préparation contenant un flavonoïde, caractérisé en ce qu'on mélange à sec le flavonoïde et un peptide hydrophile de poids moléculaire compris entre 100 D et 10⁸ D et on pétrit le mélange avec un solvant organique volatil du groupe: méthanol, éthanol, isopropanol, acétone ; ainsi que des mélanges de ces solvants, puis on le sèche.

33. Procédé suivant l'une des revendications 16 à 32, caractérisé en ce qu'on utilise comme flavonoïde un extrait de Gingko biloba.

34. Procédé suivant l'une quelconque des revendications 16 à 32, caractérisé en ce qu'on utilise comme flavonoïde la rutine.

35. Utilisation d'une préparation de flavonoïde suivant l'une des revendications 1 à 15 pour la production d'une préparation pharmaceutique.

36. Utilisation d'une préparation de flavonoïde suivant l'une des revendications 1 à 15 pour la production d'une préparation cosmétique.

37. Utilisation d'une préparation de flavonoïde suivant l'une des revendications 1 à 15 pour la production d'un aliment diététique (health care).

38. Utilisation d'une préparation de flavonoïde suivant l'une des revendications 1 à 15 contenant de la rutine comme flavonoïde pour l'obtention d'un médicament destiné à produire l'effet pharmacologique de la quercétine.

39. Utilisation d'une préparation de flavonoïde suivant l'une des revendications 1 à 15 contenant de la rutine comme flavonoïde pour l'obtention d'un médicament destine à produire l'effet pharmacologique de l'huile de camphre.

40. Utilisation d'une préparation de flavonoïde suivant l'une des revendications 1 à 15 contenant de la rutine comme flavonoïde pour l'obtention d'un médicament destiné à produire l'effet pharmacologique de l'isorhamnétine.

41. Utilisation d'une préparation de flavonoïde suivant l'une des revendications 1 à 15 contenant de la rutine comme flavonoïde pour l'obtention d'un médicament destiné à produire l'effet pharmacologique d'un mélange de quercétine, d'huile de camphre et d'isorhamnétine.

42. Utilisation d'une préparation de flavonoïde suivant l'une des revendications 1 à 15 contenant de la rutine comme flavonoïde pour l'obtention d'un médicament destiné à produire les effets pharmacologiques les plus importants de l'extrait de Gingko biloba.
